# EUROPÄISCHE PATENTSCHRIFT

(11) **EP 3 787 494 B1**
(45) Veröffentlichungstag und Bekanntmachung des Hinweises auf die Patenterteilung: **01.01.2025**
(21) Anmeldenummer: 19732938.6
(22) Anmeldetag: 17.06.2019
(51) Int. Cl.: A61B 5/374

(54) **VORRICHTUNGEN ZUR BEREITSTELLUNG EINES PARAMETERS, DER AUF EINE ERHÖHTE WAHRSCHEINLICHKEIT DES AUFTRETENS EINES POSTOPERATIVEN DELIRS HINWEIST**
DEVICES FOR PROVIDING A PARAMETER THAT INDICATES A HIGHER LIKELIHOOD OF A POSTOPERATIVE DELIRIUM OCCURRING
DISPOSITIFS PERMETTANT D'OBTENIR UN PARAMÈTRE QUI INDIQUE UNE PROBABILITÉ ÉLEVÉE D'APPARITION D'UN DÉLIRE POSTOPÉRATOIRE

(30) Priorität: 15.06.2018 DE 102018114364
(43) Veröffentlichungstag der Anmeldung: 10.03.2021
(73) Patentinhaber: Covidien LP, Boulder CO 80301 (US)
(72) Erfinder: KOCH, Susanne, 13465 Berlin (DE); SPIES, Claudia, 10439 Berlin (DE)
(74) Vertreter: Marks & Clerk LLP
(86) Internationale Anmeldenummer: PCT/EP2019/065832
(87) Internationale Veröffentlichungsnummer: WO 2019/238974

(56) Entgegenhaltungen:
- US-A- 4 846 190
- US-A- 4 846 190
- DEINER STACIE ET AL: "Can Intraoperative Processed EEG Predict Postoperative Cognitive Dysfunction in the Elderly?", CLINICAL THERAPEUTICS, ELSEVIER, AMSTERDAM, NL, vol. 37, no. 12, 24 November 2015 (2015-11-24), pages 2700 - 2705, XP029360550, ISSN: 0149-2918, DOI: 10.1016/J.CLINTHERA.2015.11.004
- P. L. PURDON ET AL: "Electroencephalogram signatures of loss and recovery of consciousness from propofol", PROCEEDINGS OF THE NATIONAL ACADEMY OF SCIENCES, vol. 110, no. 12, 19 March 2013 (2013-03-19), pages E1142 - E1151, XP055137137, ISSN: 0027-8424, DOI: 10.1073/pnas.1221180110
- SMITH ET AL: "Anesthetic Technique (Sufentanil Versus Ketamine Plus Midazolam) and Quantitative Electroencephalographic Changes After Cardiac Surgery", JOURNAL OF CARDIOTHORACIC AND VASCULAR ANESTHESIA, ELSEVIER, AMSTERDAM, NL, vol. 20, no. 4, 1 August 2006 (2006-08-01), pages 520 - 525, XP005581306, ISSN: 1053-0770, DOI: 10.1053/J.JVCA.2005.11.014
- GU YCHEN JLU YPAN S: "Integrative Frequency power of EEG correlates with progression of mild cognitive impairment to dementia in Parkinson's disease", CLIN EEG NEUROSCI., vol. 47, no. 2, 2016, pages 113 - 117
- KONSTANZE PLASCHKE ET AL: "Early postoperative delirium after open-heart cardiac surgery is associated with decreased bispectral EEG and increased cortisol and interleukin-6", INTENSIVE CARE MEDICINE, SPRINGER, BERLIN, DE, vol. 36, no. 12, 6 August 2010 (2010-08-06), pages 2081 - 2089, XP019862083, ISSN: 1432-1238, DOI: 10.1007/S00134-010-2004-4
- DEINER STACIE ET AL: "Can Intraoperative Processed EEG Predict Postoperative Cognitive Dysfunction in the Elderly?", CLINICAL THERAPEUTICS, EXCERPTA MEDICA, PRINCETON, NJ, US, vol. 37, no. 12, 24 November 2015 (2015-11-24), pages 2700 - 2705, XP029360550, ISSN: 0149-2918, DOI: 10.1016/J.CLINTHERA.2015.11.004
- P. L. PURDON ET AL: "Electroencephalogram signatures of loss and recovery of consciousness from propofol", PROCEEDINGS OF THE NATIONAL ACADEMY OF SCIENCES, vol. 110, no. 12, 19 March 2013 (2013-03-19), pages E1142 - E1151, XP055137137, ISSN: 0027-8424, DOI: 10.1073/pnas.1221180110
- SMITH ET AL: "Anesthetic Technique (Sufentanil Versus Ketamine Plus Midazolam) and Quantitative Electroencephalographic Changes After Cardiac Surgery", JOURNAL OF CARDIOTHORACIC AND VASCULAR ANESTHESIA, SAUNDERS, PHILADELPHIA, PA, US, vol. 20, no. 4, 6 April 2006 (2006-04-06), pages 520 - 525, XP005581306, ISSN: 1053-0770, DOI: 10.1053/J.JVCA.2005.11.014
- KONSTANZE PLASCHKE ET AL: "Early postoperative delirium after open-heart cardiac surgery is associated with decreased bispectral EEG and increased cortisol and interleukin-6", INTENSIVE CARE MEDICINE, SPRINGER, BERLIN, DE, vol. 36, no. 12, 6 August 2010 (2010-08-06), pages 2081 - 2089, XP019862083, ISSN: 1432-1238, DOI: 10.1007/S00134-010-2004-4

## Beschreibung

Die Erfindung betrifft Vorrichtungen zur Bereitstellung eines Parameters, der auf eine erhöhte Wahrscheinlichkeit des Auftretens eines postoperativen Delirs hinweist.

Das postoperative Delir (POD) ist eine häufige postoperative Komplikation vor allem älterer Menschen, die zu einer verlängerten Liegedauer im Krankenhaus, Verlusten kognitiver Fähigkeiten, einer Verschlechterung der Lebensqualität nach der Operation sowie zu einer Erhöhung der Mortalität bis zu einem Jahr nach der Operation führt. Eine Vorhersage, welche Patienten ein erhöhtes Risiko für die Entwicklung eines POD haben, erfolgt anhand der Auswertung von Risikoprofilen (höheres Alter, verminderte kognitive Fähigkeiten präoperativ, etc.). Die Erstellung eines Risikoprofiles ist jedoch wenig spezifisch und wird dem tatsächlichen, individuellen Risiko der einzelnen Patienten nicht gerecht.

Die Ableitung von Hirnströmen mittels Oberflächenelektroden im Rahmen einer Elektroenzephalographie (EEG) wurde Anfang des vorigen Jahrhunderts erstmals beschrieben. Die EEG Oszillationen wurden anfänglich visuell ausgewertet, wobei vor allem epileptisches Potential, Schlafstadien-Einteilungen und Komatiefe-Beurteilung bis hin zum Nachweis des Hirntods im Fokus standen. Durch Einführung computerbasierter EEG Analysesoftware sind Auswertung und Aussagekraft der Befunde deutlich verbessert worden. Die Nutzung von EEG-Daten für eine Narkosesteuerung stellt eine übliche Maßnahme dar.

In der Elektroenzephalografie unterscheidet man verschiedene EEG Frequenzbänder. Im Rahmen der vorliegenden Patentanmeldung werden folgende Definitionen für die EEG Frequenzbänder verwendet: Direct Current EEG: < 0.5Hz; Sub-Delta-Band: 0.5-1.5Hz; Delta-Band: 1.5 bis 4Hz; Theta-Band: 4 bis <8Hz; Alpha-Band: 8 bis 12Hz; Beta-Band: > 12 bis 35Hz; Gamma-Band: > 35Hz.

Es ist des Weiteren bekannt, die individuelle Alpha-Frequenz durch Bestimmung der sogenannten Alpha-Peak Frequenz (aFP) zu charakterisieren. Hierbei handelt es sich um die Frequenz im Alpha-Band mit der höchsten Power, d.h. im Leistungsspektrum eines EEG-Signals um die Frequenz, bei der die Leistung bzw. das Amplitudenquadrat im Alpha-Band maximal ist. Gu Y, Chen J, Lu Y, Pan S.: "Integrative Frequency power of EEG correlates with progression of mild cognitive impairment to dementia in Parkinson's disease", Clin EEG Neurosci. 2016; 47 (2): 113-117, berichten, dass die Alpha-Peak Frequenz mit kognitiven Fähigkeiten korreliert.

F. M. Radtke, M. Franck, J. Lendner, S. Krüger, K. D. Wernecke, C. D. Spies: "Monitoring depth of anaesthesia in a randomized trial decreases the rate of postoperative delirium but not postoperative cognitive dysfunction", BJA: British Journal of Anaesthesia, Volume 110, Issue suppl_1, 1 June 2013, Pages i98-i105, beschreiben, dass die postoperative Delir-Häufigkeit durch ein EEG-gestütztes Neuromonitoring der Narkosetiefe signifikant gesenkt werden kann. Es wird dabei die Narkosetiefe mittels einer EEG-Auswertung gemessen, um die Anästhesie präziser ausführen und dadurch die postoperative Delir-Häufigkeit reduzieren zu können. US4846190 offenbart die Bestimmung eines Komplementes einer Wahrscheinlichkeit neurologischer Komplikationen und verwendet dazu die Abweichung der spektralen integralen Leistung des Alphabandes des EEG von einem durch eine Referenzverteilung definierten Standardbereich.

Der vorliegenden Erfindung liegt die Aufgabe zu Grunde, Vorrichtungen anzugeben, die Informationen bereitstellen, die es einem/r Arzt/Ärztin bzw. Anästhesisten/Anästhesistin ermöglichen, die Wahrscheinlichkeit für das Eintreten eines postoperativen Delirs besser einzuschätzen.

Diese Aufgabe wird durch die Merkmale der unabhängigen Ansprüche gelöst.

Ausgestaltungen der Erfindung sind in den abhängigen Ansprüchen angegeben.

Danach stellt die Erfindung in einem ersten Erfindungsaspekt eine Vorrichtung zur Bereitstellung eines Parameters bereit, der auf eine erhöhte Wahrscheinlichkeit des Auftretens eines postoperativen Delirs hinweist. Es wird mindestens ein EEG-Signal am Kopf des Patienten erfasst. Es ist vorgesehen, dass die intraoperative Alpha-Peak Frequenz des EEG-Signals bestimmt wird, wobei die Alpha-Peak Frequenz im Leistungsspektrum des EEG-Signals die Frequenz im Alpha-Band ist, bei der die Leistung am größten ist. Es wird des Weiteren geprüft, ob die bestimmte intraoperative Alpha-Peak Frequenz signifikant (d.h. in definierter Weise) niedriger liegt als ein vorgegebener Referenzwert der Alpha-Peak Frequenz. Wie noch ausgeführt werden wird, handelt es sich bei dem Referenzwert beispielsweise um eine gemittelte Alpha-Peak Frequenz einer Referenzgruppe von Patienten, die kein postoperatives Delir entwickelt hat.

Für den Fall, dass die intraoperative Alpha-Peak Frequenz signifikant niedriger liegt als der vorgegebene Referenzwert, wird eine entsprechende Information als Parameter bereitgestellt, wobei der Parameter eine erhöhte Wahrscheinlichkeit des Auftretens eines postoperativen Delirs anzeigt. Der Parameter kann durch eine/n Arzt/Ärztin in Verbindung mit weiteren Parametern für die Entscheidung genutzt werden, ob der/die Patient/Patientin frühzeitig therapeutisch behandelt wird.

Es wird darauf hingewiesen, dass der Schritt des Prüfens, ob die intraoperative Alpha-Peak Frequenz signifikant niedriger liegt als ein vorgegebener Referenzwert des Alpha-Peak, eine Differenzbildung zwischen dem vorgegebenen Referenzwert und der intraoperativen Alpha-Peak Frequenz umfasst. Sofern diese Differenz bestimmte Kriterien erfüllt, z.B. größer ist als ein vorgegebener Wert, liegt die intraoperative Alpha-Peak Frequenz "signifikant" niedriger als ein vorgegebener Referenzwert. Weiter wird darauf hingewiesen, dass der Parameter, der eine erhöhte Wahrscheinlichkeit des Auftretens eines postoperativen Delirs anzeigt, eben darin besteht anzuzeigen, dass die intraoperative Alpha-Peak Frequenz signifikant niedriger liegt als der vorgegebene Referenzwert.

Es wird weiter darauf hingewiesen, dass die Prüfung, ob die bestimmte intraoperative Alpha-Peak Frequenz signifikant niedriger liegt als ein vorgegebener Referenzwert, gemäß einer Ausführungsvariante der Erfindung über maschinelles Lernen erfolgen kann, wobei ein künstliches System aus Beispielen für eine signifikant niedrigere intraoperative Alpha-Peak Frequenz lernt und diese Beispiele nach Beendigung der Lernphase verallgemeinern kann, wobei Muster und Gesetzmäßigkeiten in den Lerndaten erkannt werden.

Die Erfindung beruht auf der überraschenden Erkenntnis, dass mit der intraoperativ zu bestimmenden Alpha-Peak Frequenz bzw. deren Abweichung von einem Referenzwert ein Parameter bereitgestellt werden kann, mit dem die Entwicklung eines postoperativen Delirs vorhergesagt oder zumindest besser eingeschätzt werden kann. Dies ermöglicht eine frühzeitige therapeutische Intervention, woraufhin die zum Teil schwerwiegenden Folgekomplikationen bei Patienten, die unter einem postoperativen Delir leiden, verhindert oder reduziert werden könnten.

Da es sich hier um eine gerätebasierte Untersuchung handelt, die auch im klinischen Alltag einfach umzusetzen ist, kann durch die Erfindung die Diagnosesicherheit deutlich erhöhen werden. Es kann gemäß der Erfindung schon intraoperativ zu einer Warnung kommen, so dass unterstützende therapeutische Maßnahmen eingeleitet werden können. Auch im Aufwachraum und der weiter betreuenden Station kann der Patient entsprechend behandelt bzw. der weitere Verlauf kann sorgfältig überwacht werden.

Eine Ausgestaltung der Erfindung sieht vor, dass der vorgegebene Referenzwert durch Mittelung der gemessenen intraoperativen Alpha-Peak Frequenzen einer Mehrzahl von Patienten ermittelt worden ist, die kein postoperatives Delir entwickelt haben. Dabei kann vorgesehen sein, dass der Referenzwert abhängig von der Altersklasse, in der der/die Patient/Patientin sich befindet, vorgegeben wird. Es wird somit beispielsweise ein erster Referenzwert für eine erste Altersklasse (zum Beispiel von 65-70 Jahren) ermittelt, indem die an einer Mehrzahl von Patienten dieser ersten Altersklasse gemessenen intraoperativen Alpha-Peak Frequenzen gemittelt werden. Ein zweiter Referenzwert für eine zweite Altersklasse (zum Beispiel von 70-80 Jahren) wird ermittelt, indem die an einer Mehrzahl von Patienten dieser zweiten Altersklasse gemessenen intraoperativen Alpha-Peak Frequenzen gemittelt werden. Durch Wahl des Referenzwertes in Abhängigkeit von einer bestimmten Altersklasse ist die Wahrscheinlichkeit der korrekten Vorhersage des Eintretens eines postoperativen Delir verbessert. Allerdings wird darauf hingewiesen, dass eine Bestimmung des Referenzwertes abhängig von der Altersklasse nur ein Ausführungsbeispiel der Erfindung darstellt.

In weiteren Ausgestaltungen kann vorgesehen sein, dass der vorgegebene Referenzwert ermittelt wird durch Berücksichtigung der gemessenen intraoperativen Alpha-Peak Frequenzen einer Mehrzahl von Patienten, die kein postoperatives Delir entwickelt haben, sowie zusätzlich durch Berücksichtigung der gemessenen intraoperativen Alpha-Peak Frequenzen einer Mehrzahl von Patienten, die ein postoperatives Delir entwickelt haben. Hierdurch kann der typische Frequenzabstand der Alpha-Peak Frequenz zwischen Patienten, die ein postoperatives Delir entwickeln und Patienten, die kein postoperatives Delir entwickeln, bei der Bestimmung des Referenzwertes berücksichtigt werden.

Eine weitere Ausgestaltung der Erfindung sieht vor, dass die intraoperativen Alpha-Peak Frequenz zu einem Zeitpunkt bestimmt wird, zu dem der/die Patient/Patientin sich in einer stabilen Narkose befindet. Beispielsweise wird die intraoperative Alpha-Peak Frequenz 15 Minuten nach Intubation oder Eintritt der Bewusstlosigkeit gemessen. Diese Werte sind jedoch nur beispielhaft zu verstehen.

Es kann dabei vorgesehen sein, dass die intraoperative Alpha-Peak Frequenz des/der Patienten/Patientin über ein definiertes intraoperatives Zeitfenster mehrfach gemessen und ein Mittelwert gebildet wird.

Wie erläutert, wird geprüft, ob die intraoperative Alpha-Peak Frequenz signifikant niedriger liegt als ein vorgegebener Referenzwert der Alpha-Peak Frequenz und für diesen Fall wird eine entsprechende Information als Parameter ausgegeben. Ein signifikantes Niedriger-Liegen der Alpha-Peak Frequenz liegt beispielsweise dann vor, wenn die Differenz zwischen dem vorgegebenen Referenzwert und der intraoperative Alpha-Peak Frequenz eine definierte prozentuale Abweichung vom Referenzwert oder eine definierte absolute Differenz zwischen der intraoperativen Alpha-Peak Frequenz und dem Referenzwert übersteigt. Im ersten Fall wird beispielsweise geprüft, ob die intraoperative Alpha-Peak Frequenz mindestens X %, beispielsweise mindestens 10 % vom Referenzwert abweicht. Im zweiten Fall wird beispielsweise geprüft, ob die Frequenzdifferenz zwischen der intraoperativen Alpha-Peak Frequenz und dem Referenzwert einen vordefinierten Wert, beispielsweise 1 Hertz übersteigt.

Gemäß der Erfindung wird bevorzugt ein frontales EEG-Signal aufgenommen, also eine frontale Ableitung vorgenommen, wobei das EEG-Signal an mindestens zwei Elektroden gemessen wird, die an unterschiedlichen Orten an der Stirn des Patienten angeordnet sind. Dabei kann vorgesehen sein, dass mehrere frontale EEG-Signale aufgenommen werden, die zur Bestimmung der Alpha-Peak Frequenz gemittelt werden. Im typischerweise eingesetzten 10-20-System ist beispielsweise vorgesehen, dass Signale von Elektroden abgeleitet werden, die an den Positionen F7, F8, Fp1, Fp2 und Fpz positioniert sind.

Es kann eine bipolare Ableitung (Differenz zweier aktiver Elektroden) oder eine unipolare Ableitung (Differenz mehrerer aktiver Elektroden gegen eine gemeinsame Referenz) erfolgen.

Eine weitere Ausgestaltung sieht vor, dass die intraoperative Alpha-Peak Frequenz an dem EEG-Signal bestimmt wird, nachdem dieses durch einen Bandpass-Filter gefiltert worden ist. Der Bandpass-Filter ist beispielsweise derart ausgebildet, dass er nur Signale des Frequenzbereichs von 0,5 - 40 Hz passieren lässt.

Eine weitere Ausgestaltung sieht vor, dass beim Bestimmen der intraoperativen Alpha-Peak Frequenz des EEG-Signals die Frequenz bestimmt wird, bei der die Leistung im Leistungsspektrum des EEG-Signals im Alpha-Band und Theta-Band am größten ist. Zwar liegt die Alpha-Peak Aktivierung intraoperativ üblicherweise im Alpha-Band. Jedoch kann bei Patienten, die nach der Operation ein postoperatives Delir entwickeln (POD Patienten) der Peak der Frequenz auch im oberen Theta-Band liegen, wobei das Theta-Band sich zwischen 4 und 8 Hz erstreckt. Auch ein solcher, sich im oberen Theta-Band liegender Peak der Frequenz wird als Alpha-peak im Sinne der vorliegenden Erfindung bezeichnet.

Die Erfindung stellt in einem zweiten Erfindungsaspekt eine Vorrichtung zur Bereitstellung eines weiteren Parameters bereit, der auf eine erhöhte Wahrscheinlichkeit des Auftretens eines postoperativen Delirs hinweist. Gemäß dem Verfahren wird mindestens ein EEG-Signal am Kopf des/der Patienten/Patientin erfasst. Es ist vorgesehen, dass die Leistung des Alpha-Bands des EEG-Signals bestimmt wird, wobei die Leistung des Alpha-Bands im Leistungsspektrum des EEG-Signals definiert ist als Integral der Leistung über alle Frequenzen im Alpha-Band. Bestimmt wird also die Leistung des EEG-Signals über alle Frequenzen im Alpha-Band.

So ist vorgesehen, dass eine erste Leistung des Alpha-Bands bestimmt wird zu einem präoperativen Zeitpunkt, der vor der Gabe eines narkoseinduzierenden Medikaments liegt, und eine zweite Leistung des Alpha-Bands bestimmt wird zu einem intraoperativen Zeitpunkt, der nach dem Eintreten der narkoseinduzierten Bewusstlosigkeit liegt. Anschließend wird geprüft, ob eine Zunahme der Leistung des Alpha-Bands von der ersten Leistung zu der zweiten Leistung unterhalb eines vordefinierten Maßes liegt. Für diesen Fall wird eine entsprechende Information als Parameter, der eine erhöhte Wahrscheinlichkeit des Auftretens eines postoperativen Delirs anzeigt, bereitgestellt.

Der zweite Erfindungsaspekt geht zunächst aus von der Grundtatsache, dass während einer Narkose die Leistung des Alpha-Bands zunimmt. Dies hängt damit zusammen, dass es durch die intraoperative Aktivierung der GABA Neuronen im frontalen Bereich zu einer Alpha-Aktivierung kommt. Die Erfindung hat nun den überraschenden Zusammenhang aufgedeckt, dass die Leistung des Alpha-Bands bei Patienten/Patientinnen, die ein postoperatives Delir entwickeln, unter Narkose in geringerem Maße ansteigt als bei Patienten/Patientinnen, die kein postoperatives Delir entwickeln. Mit anderen Worten liegt bei Patienten/Patientinnen, die ein postoperatives Delir entwickeln, die Zunahme der Leistung des Alpha-Bands von der ersten Leistung (präoperativ) zu der zweiten Leistung (intraoperativ) unterhalb eines vordefinierten Maßes (was in Extremfällen auch mit einschließt, dass die Leistung des Alpha-Bandes von präoperativ zu intraoperativ sogar abfällt).

Es wird darauf hingewiesen, dass die Prüfung, ob eine Zunahme der Leistung des Alpha-Bands von der ersten Leistung zu der zweiten Leistung unterhalb eines vordefinierten Maßes liegt, gemäß einer Ausführungsvariante der Erfindung über maschinelles Lernen erfolgen kann, wobei ein künstliches System aus Beispielen für eine reduzierte Zunahme der Leistung des Alpha-Bands lernt und diese Beispiele nach Beendigung der Lernphase verallgemeinern kann, wobei Muster und Gesetzmäßigkeiten in den Lerndaten erkannt werden.

Auch für den zweiten Erfindungsaspekt gilt, dass, da es sich um eine gerätebasierte Untersuchung handelt, die auch im klinischen Alltag einfach umzusetzen ist, die Diagnosesicherheit deutlich erhöht werden kann. Es kann schon intraoperativ zu einer Warnung kommen, so dass unterstützende therapeutische Maßnahmen eingeleitet werden können. Auch im Aufwachraum und der weiter betreuenden Station kann der/ die Patient/Patientin entsprechend behandelt bzw. der weitere Verlauf kann sorgfältig überwacht werden.

Der zweite Erfindungsaspekt kann unabhängig von dem ersten Erfindungsaspekt oder in Kombination mit diesem realisiert werden.

Eine Ausgestaltung des zweiten Erfindungsaspekts sieht vor, dass das vordefinierte Maß, unterhalb dessen eine Zunahme der Leistung des Alpha-Bands von der ersten Leistung zu der zweiten Leistung liegen muss, damit die Information bereitgestellt wird, dadurch bestimmt wird, dass
- für eine Mehrzahl von Patienten/Patientinnen, die kein postoperatives Delir entwickelt haben, die gemittelte erste Leistung des Alpha-Bands mit der gemittelten zweiten Leistung des Alpha-Bands verglichen wird, wobei ein erster Referenzwert gebildet wird,
- für eine Mehrzahl von Patienten/Patientinnen, die ein postoperatives Delir entwickelt haben, die gemittelte erste Leistung des Alpha-Bands mit der gemittelten zweiten Leistung des Alpha-Bands verglichen wird, wobei ein zweiter Referenzwert gebildet wird,
- das vordefinierte Maß auf der Grundlage der Differenz oder eines Verhältnisses zwischen dem ersten Referenzwert und dem zweiten Referenzwert gebildet wird.

Die Bewertung der Differenz der Leistung des Alpha-Bands eines/r betrachteten Patienten/Patientin zwischen präoperativ und intraoperativ dahingehend, ob eine Kondition vorliegt, bei der die Wahrscheinlichkeit des Auftretens eines postoperativen Delirs erhöht ist, wird somit auf der Grundlage von Referenzwerten vorgenommen, auf deren Grundlage das insofern relevante "vordefinierte Maß" gebildet wird. Die Referenzwerte werden an Patienten/Patientinnen ermittelt, die kein postoperatives bzw. ein postoperatives Delir entwickelt haben.

Der Vergleich der gemittelten ersten Leistung des Alpha-Bands mit der gemittelten zweiten Leistung des Alpha-Bands zur Bildung des ersten und zweiten Referenzwertes kann dabei z.B. dadurch erfolgen, dass die Differenz oder ein Quotient dieser Werte gebildet wird.

Eine Ausgestaltung sieht vor, dass das vordefinierte Maß durch die Differenz zwischen dem ersten Referenzwert und dem zweiten Referenzwert zuzüglich eines prozentualen oder absoluten Toleranzwertes gebildet wird. Der prozentuale Toleranzwert kann dabei z.B. der Standardabweichung entsprechen.

Eine weitere Ausgestaltung sieht vor, dass das vordefinierte Maß abhängig von der Altersklasse, in der der/die Patient/Patientin sich befindet, bestimmt wird. Beispielsweise werden für beide Gruppen Referenzwerte für eine erste Altersklasse (zum Beispiel von 65-70 Jahren) ermittelt und Referenzwerte für eine zweite Altersklasse (zum Beispiel von 70-80 Jahren) ermittelt. Für einen betrachteten Patienten/Patientin werden die Referenzwerte seiner Altersklasse herangezogen, so dass die Wahrscheinlichkeit der korrekten Vorhersage des Eintretens eines postoperativen Delirs verbessert ist.

Eine weitere Ausgestaltung sieht vor, dass die erste Leistung des Alpha-Bands und die zweite Leistung des Alpha-Bands sowie die Zunahme der Leistung des Alpha-Bands von der ersten Leistung zu der zweiten Leistung in dB bestimmt werden, und dass das Maß, unterhalb dessen eine Zunahme der Leistung des Alpha-Bands von der ersten Leistung zu der zweiten Leistung liegen muss, damit die Information bereitgestellt wird und geprüft wird, ebenfalls in dB angegeben wird. Entsprechende Werte der Leistung in dB werden von modernen EEG-Narkosemonitoren direkt ausgegeben und sind somit in einfacher Weise zu erhalten.

Ein Ausführungsbeispiel sieht vor, dass die Zunahme der Leistung des Alpha-Bands von der ersten Leistung zu der zweiten Leistung unterhalb von 15 dB, insbesondere unterhalb von 12 dB liegen muss, damit die Information bereitgestellt bzw. der Parameter erzeugt wird.

Auch bei dem zweiten Erfindungsaspekt gilt, dass die Leistung des Alpha-Bands gemäß einer Ausgestaltung an einem EEG-Signal bestimmt wird, nachdem dieses durch einen Bandpass-Filter gefiltert worden ist. Es wird bevorzugt mindestens ein frontales EEG-Signal am Kopf des/der Patienten/Patientin erfasst, wobei vorgesehen sein kann, dass mehrere frontale EEG-Signale des/der Patienten/Patientin erfasst werden, die zur Bestimmung der Leistung des Alpha-Bands gemittelt werden. Beispielsweise erfolgt eine Ableitung des Signals an Elektroden, die an den Positionen F7, F8, Fp1, Fp2 und Fpz im 10-20-System positioniert sind.

Die Erfindung stellt in einem dritten Erfindungsaspekt eine Vorrichtung zur Bereitstellung eines weiteren Parameters bereit, der auf eine erhöhte Wahrscheinlichkeit des Auftretens eines postoperativen Delirs hinweist. Gemäß dem Verfahren wird mindestens ein EEG-Signal am Kopf des/der Patienten/Patientin erfasst, wobei nur der Frequenzbereich des EEG-Signals kleiner als 0,5 Hz erfasst bzw. betrachtet wird. Signale in diesem Frequenzbereich werden auch als "Direct Current EEG" oder "DC-EEG" bezeichnet, da die Änderung des Signals sehr langsam ist. Gemäß einer Ausgestaltung werden dabei sämtliche Signale mit einer Frequenz kleiner 0,5 Hz erfasst. In einer alternativen Ausgestaltung kann der betrachtete Frequenzbereich weiter eingeschränkt werden, beispielsweise auf Signale mit einer Frequenz kleiner 0,1 Hz.

Gemäß der Vorrichtung des dritten Erfindungsaspekts wird die mittlere Amplitude des EEG-Signals in einem aktuellen Zeitfenster des EEG-Signals bestimmt. Das betrachtete Zeitfenster betrachtet das EEG-Signal in einem Zeitraum, der von der aktuellen Zeit eine definierte Zeitspanne überspannt, beispielsweise ein EEG-Signal über den Zeitraum von zwei Minuten oder einer Minute oder über die letzten 30, 20, 10 oder 2 Sekunden. Insofern handelt es sich um ein mit der Zeit wanderndes Zeitfenster bzw. die Bestimmung einer Amplituden Messung über die Zeit. Die Bestimmung der mittleren Amplitude kann fortlaufend oder in bestimmten Intervallen erfolgen.

Es wird der Verlauf der mittleren Amplitude des EEG-Signals zwischen einem präoperativen Zeitpunkt, der vor der Gabe eines narkoseinduzierenden Medikaments liegt, und einem intraoperativen Zeitpunkt, der nach dem Eintreten der narkoseinduzierten Bewusstlosigkeit liegt, bestimmt. Dabei wird insbesondere die mittlere Amplitude des EEG-Signals beim Eintritt der narkoseinduzierten Bewusstlosigkeit bestimmt. Das Eintreten des Bewusstseinsverlusts bei der Narkose ("loss of consciousness" - LOC) kann beispielsweise über den Lidschlussreflex genau erfasst werden.

Weiter wird geprüft, ob eine Zunahme der mittleren Amplitude des EEG-Signals beim Eintritt der narkoseinduzierten Bewusstlosigkeit oberhalb eines vordefinierten Maßes liegt. Für diesen Fall wird eine entsprechende Information als Parameter, der eine erhöhte Wahrscheinlichkeit des Auftretens eines postoperativen Delirs anzeigt, bereitgestellt bzw. ausgegeben.

Anschließend, d. h. nach Eintritt der narkoseinduzierten Bewusstlosigkeit, fällt die mittlere Amplitude wieder ab, so dass die mittlere Amplitude des EEG-Signals beim Eintritt der narkoseinduzierten Bewusstlosigkeit einen Peak aufweist und dementsprechend messtechnisch leicht erfasst werden kann.

Dieser dritte Erfindungsaspekt beruht auf der überraschenden Erkenntnis, dass Patienten/Patientinnen, bei denen zum Zeitpunkt des Eintretens des Bewusstseinsverlusts die mittlere Amplitude des "Direct Current" EEG-Signals (also des Signalanteils des EEG-Signals mit einer Frequenz kleiner als 0,5 Hz) einen signifikanten Anstieg erfährt, mit signifikant größerer Wahrscheinlichkeit ein postoperatives Delir entwickeln. Bei Patienten/Patientinnen, die kein postoperatives Delir entwickeln, tritt dagegen kein Anstieg oder kein signifikanter Anstieg der mittleren Amplitude des "Direct Current" EEG-Signals zum Zeitpunkt des Eintretens des Bewusstseinsverlustes auf.

Der dritte Erfindungsaspekt kann unabhängig von dem ersten und zweiten Erfindungsaspekt oder in Kombination mit einem oder beiden von diesen realisiert werden.

Zur Erfassung des "Direct Current" EEG-Signals kann vorgesehen sein, dass das EEG-Signal einen Tiefpassfilter mit einer Eckfrequenz von 0,5 Hz durchläuft.

Gemäß einer Ausgestaltung des dritten Erfindungsaspekts ist vorgesehen, dass das vordefinierte Maß, oberhalb dessen eine Zunahme der mittleren Amplitude des EEG-Signals beim Eintritt der narkoseinduzierten Bewusstlosigkeit liegen muss, damit eine Information bereitgestellt wird, dadurch bestimmt wird, dass für eine Mehrzahl von Patienten/Patientinnen, die kein postoperatives Delir entwickelt haben, die mittleren Amplitude des EEG-Signals beim Eintritt der narkoseinduzierten Bewusstlosigkeit bestimmt wird, wobei ein erster Referenzwert gebildet wird. Weiter wird für eine Mehrzahl von Patienten/Patientinnen, die ein postoperatives Delir entwickelt haben, die mittlere Amplitude des EEG-Signals beim Eintritt der narkoseinduzierten Bewusstlosigkeit bestimmt, wobei ein zweiter Referenzwert gebildet wird. Das vordefinierte Maß wird auf der Grundlage der Differenz oder eines Verhältnisses zwischen dem ersten Referenzwert und dem zweiten Referenzwert gebildet. Beispielsweise kann vorgesehen sein, dass der zweite Referenzwert um mindestens den Faktor 3, insbesondere um mindestens den Faktor 5, insbesondere um mindestens den Faktor 10 größer sein muss als der erste Referenzwert, damit das vorgegebene Maß überschritten wird.

Dabei kann vorgesehen sein, dass das vordefinierte Maß durch die Differenz zwischen dem ersten Referenzwert und dem zweiten Referenzwert abzüglich eines prozentualen oder absoluten Toleranzwertes gebildet wird. Weiter kann vorgesehen sein, dass das vordefinierte Maß abhängig von der Altersklasse, in der der Patient sich befindet, bestimmt wird.

Die Ableitung des EEG-Signals erfolgt bei dem dritten Erfindungsaspekt gemäß einer Ausführungsvariante an einer Elektrode, die an der Position Cz (also zentral) im 10-20-System positioniert ist, und an einer Referenzelektrode z.B. am Ohr. Für diese zentrale EEG-Ableitung wurden experimentell die besten Signale erfasst. Alternativ kann die Ableitung jedoch auch frontal erfolgen, beispielsweise wiederum über Elektroden, die an den Positionen F7, F8, Fp1, Fp2 und Fpz positioniert sind.

Die Erfindung wird definiert mittels der unabhängigen Ansprüche.

Die Vorrichtungen der Erfindung und Verfahren, die nicht Teil der beanspruchten Erfindung sind, werden nachfolgend unter Bezugnahme auf die Figuren der Zeichnung näher erläutert. Es zeigen:
- Figur 1: beispielhaft EEG-Signale im Wachzustand und nach einer narkoseinduzierten Bewusstlosigkeit jeweils sowohl als zeitabhängiges Signal und im Leistungsspektrum;
- Figur 2: beispielhaft im Leistungsspektrum die intraoperative Alpha-Peak Frequenz sowohl für eine Patientengruppe, die kein postoperatives Delir (nonPOD) entwickelt hat, als auch für eine Patientengruppe, die ein postoperatives Delir (POD) entwickelt hat;
- Figur 3: beispielhaft die Differenz zwischen der präoperativen und der intraoperativen Leistung des Alpha-Bands gemessen in dB, wobei die Leistungen des Alpha-Bands gemessen wurden sowohl für eine Patientengruppe, die kein postoperatives Delir (nonPOD) entwickelt hat, als auch für eine Patientengruppe (POD), die ein postoperatives Delir entwickelt hat;
- Figur 4: ein Ablaufdiagramm eines ersten Verfahrens zur Bereitstellung eines Parameters, der auf eine erhöhte Wahrscheinlichkeit des Auftretens eines postoperativen Delirs hinweist;
- Figur 5: ein Ablaufdiagramm eines zweiten Verfahrens zur Bereitstellung eines Parameters, der auf eine erhöhte Wahrscheinlichkeit des Auftretens eines postoperativen Delirs hinweist;
- Figur 6: beispielhaft die mittlere Amplitude des Direct Current EEG-Signals zu einem Zeitpunkt vor Einleitung einer Narkose (1), zu dem Zeitpunkt des Eintritts des Bewusstseinsverlustes (2) und zu einem Zeitpunkt nach Eintritt des Bewusstseinsverlustes (3), wobei die mittlere Amplitude für eine Patientengruppe, die ein postoperatives Delir (POD) entwickelt hat, zum Zeitpunkt des Eintritts des Bewusstseinsverlustes signifikant ansteigt;
- Figur 7: ein Ablaufdiagramm eines dritten Verfahrens zur Bereitstellung eines Parameters, der auf eine erhöhte Wahrscheinlichkeit des Auftretens eines postoperativen Delirs hinweist;
- Figur 8: beispielhaft eine Vorrichtung zur Durchführung der Verfahren der Figuren 4 und/oder 5 und/oder 7; und
- Figur 9: Positionierungspunkte für EEG-Elektroden gemäß dem 10-20 System.

Die Erfindung umfasst drei Erfindungsvarianten, wobei die eine Erfindungsvariante die Bestimmung der intraoperative Alpha-Peak Frequenz eines Patienten, die zweite Erfindungsvariante die Bestimmung der Differenz zwischen der präoperativen und der intraoperativen Leistung des Alpha-Bands eines Patienten, und die dritte Erfindungsvariante die Bestimmung der mittleren Amplitude des "Direct Current" EEG-Signals und dessen Verlauf beim Eintritt der narkoseinduzierten Bewusstlosigkeit betrachtet.

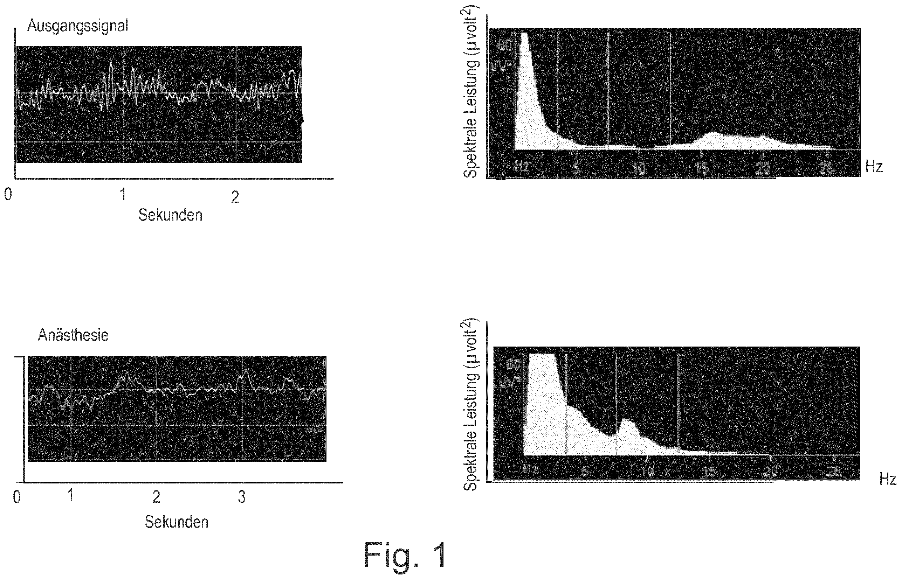

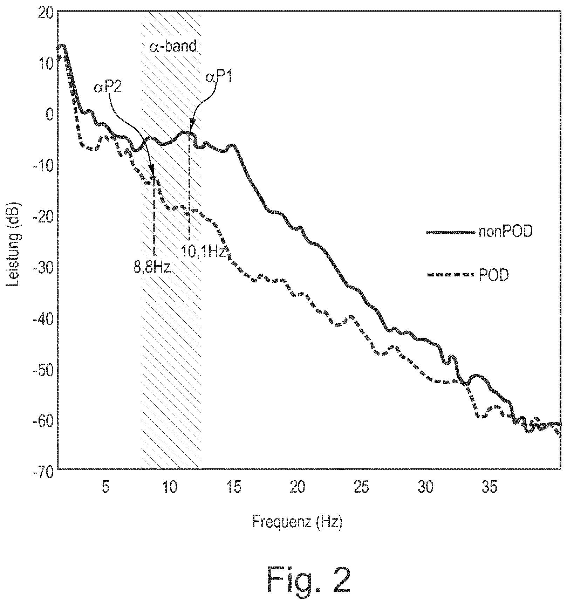

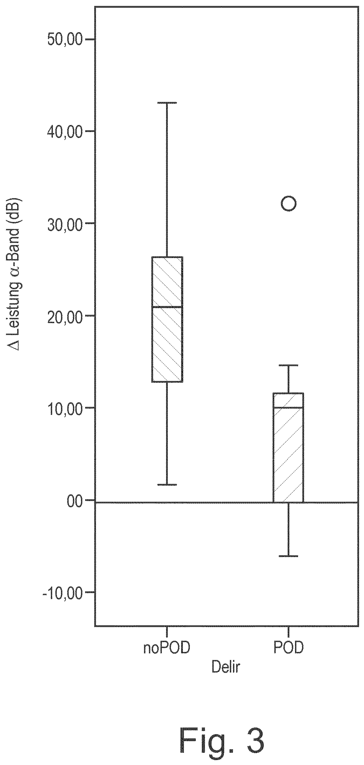

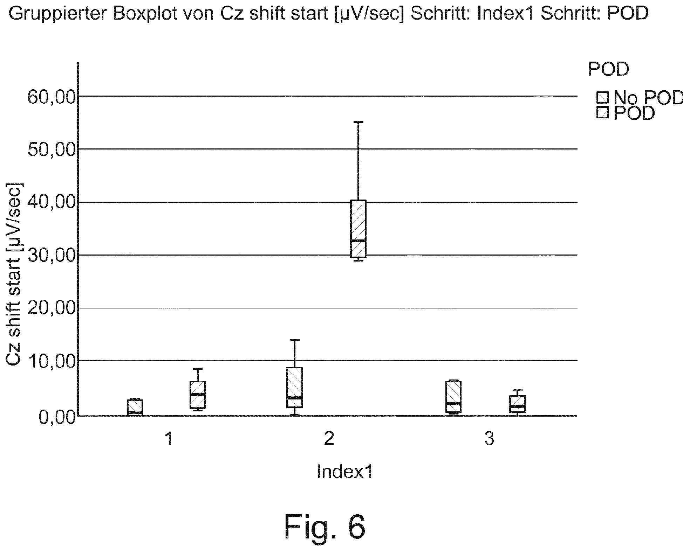

Anhand der Figuren 1 und 2 wird der gemäß der ersten Erfindungsvariante erstmals erkannte Zusammenhang erläutert, der anhand einer Studie belegt worden ist. Anhand der Figuren 1 und 3 wird der gemäß der zweiten Erfindungsvariante erstmals erkannte Zusammenhang erläutert, der anhand einer Studie belegt worden ist. Anhand der Figure 6 wird der gemäß der dritten Erfindungsvariante erstmals erkannte Zusammenhang erläutert, der anhand einer Studie belegt worden ist.

Die Figur 1 zeigt zur Erläuterung des Hintergrunds der Erfindung in der oberen Darstellung ("Baseline") ein EEG-Signal, wie es bei einem/einer Patienten/Patientin im Wachzustand auftritt. Das Signal ist sowohl als Zeitsignal (links) als auch nach einer Spektralanalyse als Leistungsspektrum (rechts) dargestellt. Im Leistungsspektrum ist die Power (das Amplitudenquadrat) in µV² gegen die Frequenz in Hz aufgetragen. Das Leistungsspektrum gibt den jeweiligen Anteil der einzelnen Frequenzbereiche am Gesamtleistungsanteil des Rohsignals wieder.

Die untere Darstellung ("Anästhesie") der Figur 1 zeigt ein EEG-Signal unter Narkose. Es ist erkennbar, dass das Frequenzspektrum insgesamt gegenüber dem Wert beim wachen Patienten/Patientin nach links verschoben ist. Im Detail verhält es sich so, dass es unter Narkose zu einem Anstieg der Frequenzen aus dem Delta-, Theta- und Alpha-Band kommt, während die Beta- und Gamma-Wellen abfallen. Dabei haben Purdon PL, Pierce ET, Mukamel EA, Prerau MJ, Walsh JL, Wong KFK, Salazar-Gomez AF, Harrell PG, Sampson AL, Cimenser A, Ching S, Kopell NJ, Tavares-SToeckel C, Habeeb K, Merhar R, Brown E.: "Electroencephalogram signatures of loss and recovery of consciousness from propofol", PNAS 2013; 110 (12): E1142-1151, gezeigt, dass es bei einer tiefen Bewusstlosigkeit induziert durch GABA aktivierende Anästhetika vor allem zu einer charakteristischen frontalen Alpha-Band Aktivierung kommt.

Die Figur 2 zeigt im Leistungsspektrum, in dem die Leistung in dB gegenüber der Frequenz auftragen ist, die intraoperative Alpha-Peak Frequenz sowohl für eine Patientengruppe, die kein postoperatives Delir entwickelt hat, als auch für eine Patientengruppe, die ein postoperatives Delir entwickelt hat. Die Alpha-Peak Frequenz ist dabei definiert als die Frequenz im Alpha-Band, bei der die Leistung am größten ist. Alternativ kann zusätzlich das Theta-Band oder der obere Teil des Theta-Bands (zwischen 6 und 8 Hz) betrachtet werden, d.h. die Alpha-Peak Frequenz ist definiert als die Frequenz im Alpha-Band und Theta-Band (bzw. oberen Bereich des Theta-Bands), bei der die Leistung am größten ist.

Dabei konnte in einer prospektiven Observationsstudie der Zusammenhang gezeigt werden, dass die intraoperative Alpha-Peak Frequenz bei Patienten/Patientinnen, die nach der Operation kein postoperatives Delir entwickeln (non-POD), höher liegt als bei Patienten/Patientinnen, die nach der Operation ein postoperatives Delir entwickeln (POD). So liegt die Alpha-Peak Frequenz α_{P1} in der Figur für non-POD Patienten/Patientinnen bei 10.1 Hertz. Bei PODPatienten/Patientinnen liegt die Alpha-Peak Frequenz α_{P2} bei 8,8 Hertz und damit signifikant niedriger. Die Standardabweichung des Werts von 10,1 Hertz lag bei 0,77 Hertz. Die Standardabweichung des Werts von 8,8 Hertz lag bei 0,87 Hertz.

Die Studie wurde an einer altersgematchten Patientengruppe mit 11 POD Patienten/Patientinnen und 11 non-POD Patienten/Patientinnen vorgenommen. Es konnte somit gezeigt werden, dass die intraoperative Alpha-Peak Frequenz bei POD Patienten/Patientinnen signifikant niedriger liegt als bei non-POD Patienten/Patientinnen.

Die Figur 3 zeigt an einem Diagramm die Differenz zwischen der präoperativen und der intraoperativen Leistung des Alpha-Bands gemessen in dB für eine non-POD Patientengruppe und für eine POD-Patientengruppe. Dabei konnte in einer prospektiven Observationsstudie der Zusammenhang gezeigt werden, dass die Leistung des Alpha-Bands von präoperativ zu intraoperativ bei POD Patienten/Patientinnen weniger stark ansteigt als bei non-POD Patienten/Patientinnen. So liegt gemäß der Figur 3 der Mittelwert der Differenz Δ von intraoperativ zu präoperativ für non-POD Patienten/Patientinnen bei etwa 21 dB. Bei POD-Patienten/Patientinnen liegt dieser Mittelwert bei ca. 10 dB und damit signifikant niedriger. Die Standardabweichung bei non-POD Patienten/Patientinnen lag bei etwa 13 dB. Die Standardabweichung bei POD Patienten/Patientinnen lag bei etwa 11,5 dB. Die Standardabweichung ist in der Figur 3 ebenfalls dargestellt.

Die Studie wurde an einer Patientengruppe mit 19 POD Patienten/Patientinnen und 35 non-POD Patienten/Patientinnen mit einem Alter über 65 Jahren durchgeführt, wobei die Narkoseeinleitung mit einem der meist gebrauchten Anästhetika, nämlich Propofol, erfolgte. Es konnte somit gezeigt werden, dass der Anstieg der Leistung des EEG-Signals im Alpha-Band von präoperativ zu intraoperativ bei POD Patienten/Patientinnen signifikant reduziert und dementsprechend die Differenz der Leistung im Alpha-Band von intraoperativ zu präoperativ bei POD Patienten/Patientinnen signifikant kleiner ist.

Die Messungen entsprechend den Figuren 2 und 3 wurden wie folgt durchgeführt:

### a) EEG Ableitung:

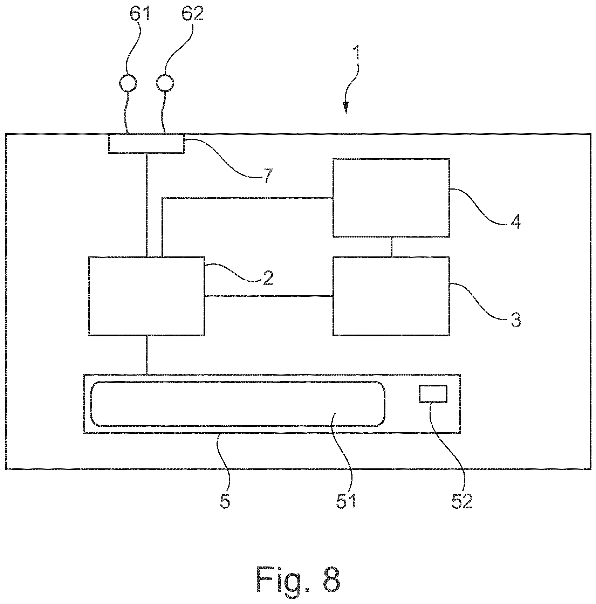

Ein kontinuierliches, intraoperatives EEG wurde mit einem EEG-basierten Gehirnfunktions-Monitor (dem "SEDLine-Monitor" der Firma Masimo Corporation, Irvine, California) von Beginn der Anästhesie bis zum Ende der Narkose aufgezeichnet. Die Oberflächen EEG Klebeelektroden (Firma Masimo, 4248RD SEDLine Sensor, Single Patient Use, Non-Sterile) wurden an den Positionen F7, F8, FP1 und FP2 entsprechend dem 10/20 System angebracht, mit Fpz als Erdelektrode und der Referenz circa 1cm oberhalb von Fpz, vgl. Figur 8. Dazu wurden die Stirn sowie die Schläfen des Patienten gründlich desinfiziert und von Hautfetten befreit. Die Impedanz der einzelnen Elektroden lag unter 5 kΩ, die Abtastrate lag bei 250Hz.

Nach dem Verbinden der Klebeelektroden mit dem EEG-basierten Gehirnfunktions-Monitor wurde mit der Ableitung und Aufzeichnung eines kontinuierlichen 4-Kanal EEG begonnen. Die Patienten/Patientinnen waren zu diesem Zeitpunkt noch wach, sodass die ersten Werte der Ableitung einer Baseline-Aktivität entsprachen. Um definierte Zeitpunkte während der EEG Ableitung zu bestimmen, wurden manuell "Event Marker" während der EEG Aufzeichnung im EEG gesetzt. Eventmarker: "Baseline" = wacher Patient/Patientin, vor Gabe von Anästhetika, "Start Anästhesie" = Beginn Anästhetika Gabe, "loss of consciousness" = fehlender Augenlidreflex, "ITN" = Intubation des/der Patienten/Patientin, "OP" = stabile intraoperative Phase 15-30min nach ITN. Alle Patienten/Patientinnen erhielten das Medikament Propofol intravenös zur Narkoseeinleitung, die Aufrechterhaltung erfolgte mit i.v. Propofol oder mit den Inhalationsanästhetika Desfluran oder Sevofluran. Die aufgezeichneten EEG-Daten wurden aus dem SEDLine Monitor exportiert.

### b) EEG Auswertung:

Die Roh-EEG-Daten wurden mit einem Bandpass-Filter von 0.5 - 40Hz (Brain Vision Analyzer Software) versehen. Anschließend erfolgte eine visuelle EEG Datenanalyse, wobei jeweils zu den Zeitpunkten "Baseline" und "OP" ein 10 Sekunden andauerndes, Artefakt-freies EEG Zeitfenster ausgewählt wurde. Die EEG Daten wurden in ein "Baseline" und ein "Intraoperatives" EEG segmentiert. Die weitere Datenanalyse erfolgte mit Hilfe der Chronux Toolbox (Bookil et al, 2010) für Matlab (The MathWorks, Inc., Natick, Massachusetts, United States). Das Power Spektrum über allen Frequenzbänder (slow und fast delta, theta, alpha, beta) wurde mittels Multitaper Methode mit 2 Sekunden Zeitfenstern, mit 1.9 Sekunden Überlappung, Time-Bandwith Produkt TW=3, Anzahl der Taper K=5 und spektraler Auflösung von 2W=3Hz berechnet. Die Berechnung erfolgte mittels digitaler, computergestützter EEG-Signalverarbeitung. Grundlage hierfür ist die Spektralanalyse des Roh-EEG mittels Fast-Fourier-Transformation, durch die für das jeweils aktuell zu analysierende Zeitfenster Leistungsanteile berechnet werden können.

Die Daten wurden anschließend in eine Dezibel Skala transformiert [*Power*(*dB*) = 10*log*10(*Power*(*µV*))]. Um die frontale EEG Power besser abbilden zu können, wurde eine gepoolte frontale Elektrode berechnet, in die die Signale der Elektroden Fp1, Fp2, F7 und F8 gleich gewichtet einflossen.

Anhand der ermittelten Spektren wurde die Peak Frequenz (Hz) im Alpha-Band (8-12Hz) (aPF) entsprechend Figur 2 bestimmt. Des Weiteren wurde die Differenz zwischen der Leistung des Signals im Alpha-Band zwischen intraoperativ und präoperativ entsprechend der Figur 3 berechnet (Differenz der Alpha-Band Power OP zu Alpha-Band Power Baseline).

### c) Delir Screening:

Nach der Operation, ab Aufnahme in den Aufwachraum erfolgte die regelmäßige Erhebung eines Delir Scorings. Das postoperative Delir wurde anhand der DSM V (Diagnostic and Statistical Manual of Mental Disorders) Kriterien definiert. Während des Aufenthaltes im Aufwachraum wurde der Nurse-Detektion Score (NuDESC) in regelmäßigen Abständen erhoben. Alle Patienten/Patientinnen mit einem NuDESC score ≥ 2 zu einem Zeitpunkt während des Aufenthaltes im Aufwachraum werden als Patient/Patientinmit postoperativem Delir (POD-Gruppe) charakterisiert, Patienten/Patientinnen mit einem NuDESC score ≤ 1 sind als Patienten/Patientinnen ohne postoperativem Delir (NonPOD-Gruppe) charakterisiert.

### d) Statistische Auswertung:

Statistische Berechnungen der Alpha-Peak Frequenz und der Differenz der Alpha-Band Power OP zur Alpha-Band Power Baseline erfolgte mit SPSS, Version 24 (Copyright SPSS, Inc., Chicago, IL 60606, USA) mittels Mann-Whitney-U-Test und Kruskal-Wallis-Test.

Die ermittelten Zusammenhänge werden erfindungsgemäß elektronisch bzw. computerbasiert ausgewertet und dazu verwendet, die intraoperative Alpha-Peak Frequenz bzw. das Maß des Anstiegs der Leistung des EEG-Signals im Alpha-Band von präoperativ zu intraoperativ bei einem/r Patienten/Patientin zu bestimmen. Das zugehörige Programm kann dabei als Softwaretool in einen EEG-basierten Gehirnfunktions-Monitor bzw. Elektroenzephalograph integriert werden.

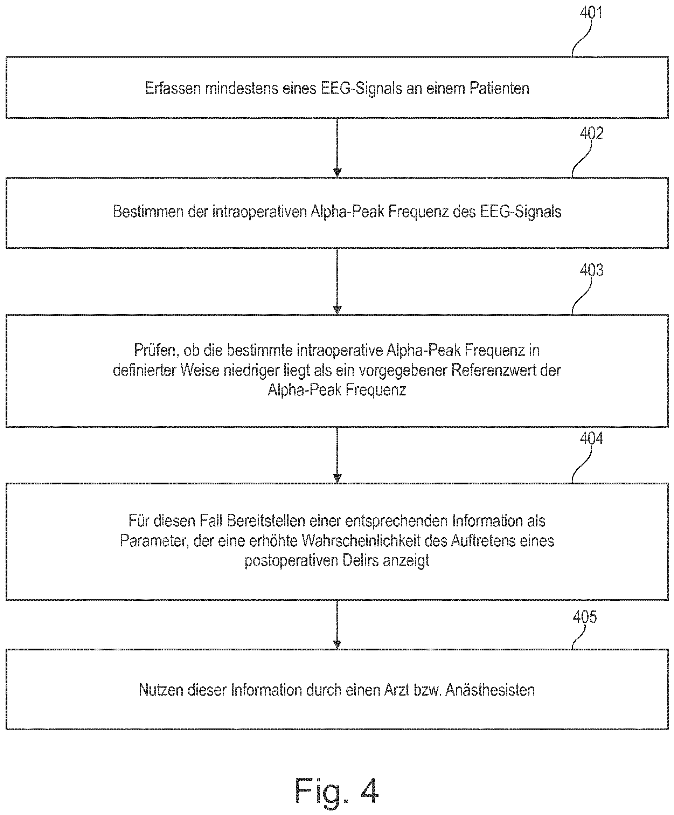

Die Figur 4 zeigt ein erstes Verfahren zur Bestimmung eines Parameters, der auf eine erhöhte Wahrscheinlichkeit des Auftretens eines postoperativen Delirs hinweist. Gemäß Schritt 401 wird mindestens ein frontales EEG-Signal an einem/r Patienten/Patientin erfasst. Beispielsweise werden vier EEG-Signale über Elektroden an den Positionen F7, F8, FP1 und FP2 entsprechend dem 10/20 System, mit Fpz als Referenzelektrode aufgenommen und es wird über diese Signale gemittelt. Eine zusätzliche Erdungselektrode liegt leicht oberhalb von Fpz.

Gemäß Schritt 402 wird anschließend die intraoperative Alpha-Peak Frequenz des EEG-Signals bestimmt. Dies erfolgt zu einem Zeitpunkt, zu dem der/die Patient/Patientin sich in einer stabilen Narkose befindet, etwa 15-30 min nach Eintritt der Bewusstlosigkeit. Als Nächstes erfolgt eine Prüfung, ob die gemessene intraoperative Alpha-Peak Frequenz signifikant niedriger liegt als ein vorgegebener Referenzwert der Alpha-Peak Frequenz. Der vorgegebene Referenzwert wurde dabei vorab ermittelt durch Mittelung der gemessenen intraoperativen Alpha-Peak Frequenzen einer Mehrzahl von Patienten, die kein postoperatives Delir entwickelt haben. Dabei ist der vorgegebene Referenzwert angepasst an die Altersklasse des Patienten ausgewählt. Die aktuell bestimmte intraoperative Alpha-Peak Frequenz liegt dabei beispielsweise dann gegenüber dem vorgegebenen Referenzwert in signifikanter Weise niedriger, wenn die Differenz zwischen dem vorgegebenen Referenzwert und der gemessenen intraoperative Alpha-Peak Frequenz eine definierte prozentuale Abweichung vom Referenzwert oder eine definierte absolute Differenz zwischen der intraoperativen Alpha-Peak Frequenz und dem Referenzwert übersteigt. Gemäß den Werten der Figur 2 liegt der Referenzwert bei 10,1 Hertz und es wird z.B. eine signifikante Abweichung angenommen, wenn der gemessene Wert der Alpha-Peak Frequenz unter 9,5 Hertz liegt.

Für diesen Fall wird gemäß Schritt 404 eine entsprechende Information als Parameter bereitgestellt, der eine erhöhte Wahrscheinlichkeit für das Auftreten eines postoperativen Delirs anzeigt.

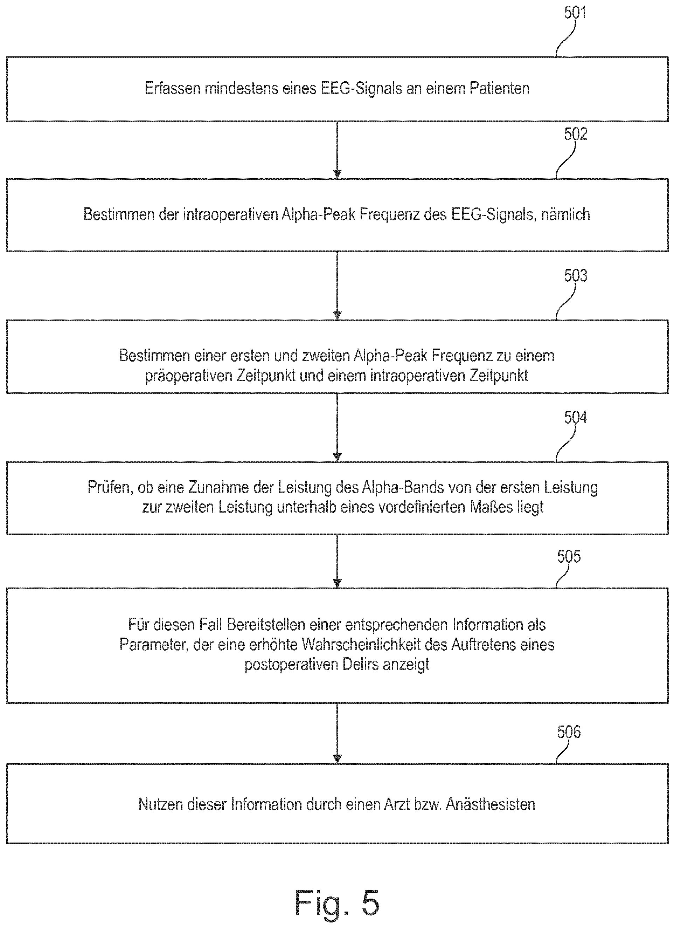

Die Figur 5 zeigt ein zweites Verfahren zur Bestimmung eines Parameters, der auf eine erhöhte Wahrscheinlichkeit des Auftretens eines postoperativen Delirs hinweist. Gemäß Schritt 501 wird wiederum mindestens ein frontales EEG-Signal an einem/r Patienten/Patientin erfasst. Beispielsweise werden vier EEG-Signale über Elektroden an den Positionen F7, F8, FP1 und FP2 entsprechend dem 10/20 System, mit Fpz als Referenzelektrode aufgenommen und es wird über diese Signale gemittelt.

Gemäß Schritt 502 wird anschließend die Leistung des Alpha-Bands des EEG-Signals bestimmt, wobei die Leistung des Alpha-Bands im Leistungsspektrum des EEG-Signals definiert ist als Integral der Leistung über alle Frequenzen im Alpha-Band. So wird gemäß Schritt 503 eine erste Leistung des Alpha-Bands zu einem präoperativen Zeitpunkt, der vor der Gabe eines narkoseinduzierenden Medikaments liegt, und eine zweite Leistung des Alpha-Bands zu einem intraoperativen Zeitpunkt, der nach dem Eintreten der narkoseinduzierten Bewusstlosigkeit liegt, bestimmt.

Anschließend wird geprüft, ob eine Zunahme der Leistung des Alpha-Bands von der ersten Leistung zu der zweiten Leistung unterhalb eines vordefinierten Maßes liegt. Das vordefinierte Maß ist beispielsweise anhand von Referenzwerten festgelegt worden, die an POD Patienten/Patientinnen und nonPOD Patienten/Patientinnen gemessen wurden. Das vordefinierte Maß kann beispielsweise ein bestimmter dB Wert sein, um den die Leistung des Alpha-Bands von präoperativ nach intraoperativ maximal zunehmen darf, damit eine Signifikanz vorliegt. Gemäß den Werten der Figur 3 liegt das vorgegebene Maß beispielsweise bei 15 dB, d.h. wenn die Leistung im Alpha-Band von präoperativ zu intraoperativ um weniger als 15 dB zunimmt, wird eine Signifikanz angenommen. In einem solchen Fall wird gemäß Schritt 505 eine entsprechende Information als Parameter, der das Eintreten eines postoperativen Delirs indiziell anzeigt, bereitgestellt.

Die Figur 6 zeigt Messwerte zur dritten Erfindungsvariante, die die Bestimmung der mittleren Amplitude des "Direct Current" EEG-Signals und dessen Verlauf beim Eintritt der narkoseinduzierten Bewusstlosigkeit betrachtet. In diesem Zusammenhang wird zunächst darauf hingewiesen, dass handelsübliche EEG-basierte Gehirnfunktions-Monitore einen Hochpassfilter aufweisen, der den Frequenzbereich des Signals kleiner 0,5 Hz herausfiltert. Davon abweichend betrachtet die dritte Erfüllungsvariante gerade den Frequenzbereich kleiner 0,5 Hz, der auch als "Direct Current" EEG oder DC-EEG bezeichnet wird. Hierzu kann beispielsweise vorgesehen sein, dass das gemessene Signal einen Tiefpassfilter mit einer Grenzfrequenz vom 0,5 Hz durchläuft.

Das gemessene DC-EEG-Signal wird fortlaufend im Hinblick auf seine Amplitude ausgewertet, wobei der Mittelwert der Amplitude in einem aktuellen, mitlaufenden Zeitfenster berechnet wird. Dieser Mittelwert der Amplitude ist zu unterscheiden von dem Mittelwert des Signals, von dem aus die Amplitude gemessen wird. Der Mittelwert des Signals wird beispielsweise in dem gleichen Zeitfenster oder über ein längeres Zeitfenster oder über die gesamte Messzeit ermittelt oder durch den EEG-basierten Gehirnfunktions-Monitor festgelegt.

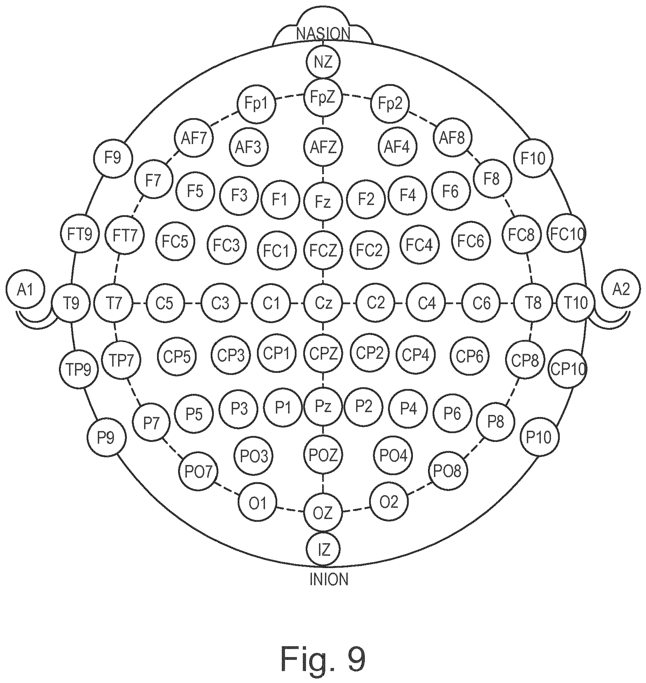

Die Figur 6 zeigt die Messung der mittleren Amplitude zu drei Zeitpunkten 1, 2, 3, wobei der Zeitpunkt 1 ein präoperativer Zeitpunkt ist, vor der Gabe eines narkoseinduzierten Medikaments, der Zeitpunkt 2 den Eintritt der narkoseinduzierten Bewusstlosigkeit bezeichnet und der Zeitpunkt 3 ein Zeitpunkt nach dem Eintreten der narkoseinduzierten Bewusstlosigkeit ist. Auf der y-Achse ist die gemittelte Amplitude aufgetragen, bezeichnet als "Cz-Shift", da die Ableitung an dem Punkt Cz im 10-20 System erfolgte (vgl. Figur 9), wobei dies nur beispielhaft zu verstehen ist.

Weiter unterscheidet die Figur 6 zwischen POD Patienten/Patientinnen und nonPOD-Patienten/Patientinnen.

Zum Zeitpunkt 1 ist die mittlere Amplitude des DC-EEG-Signals sowohl bei POD-Patienten/Patientinnen als auch beim nonPOD-Patienten/Patientinnen relativ gering. Nachfolgend sind die Werte einer Gruppenstatistik zum Zeitpunkt 1 für eine Patientengruppe mit N=7 POD-Patienten/Patientinnen und N=9 nonPOD-Patienten/Patientinnen angegeben.

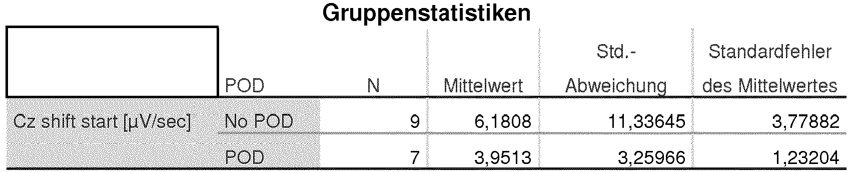

Zum Zeitpunkt 2, d. h. beim Eintritt der narkoseinduzierten Bewusstlosigkeit, ist bei den POD-Patienten/Patientinnen die mittlere Amplitude signifikant angestiegen, während sie bei den nonPOD-Patienten/Patientinnen nur geringfügig angestiegen ist. Nachfolgend sind die Werte einer Gruppenstatistik zum Zeitpunkt 2 für eine Patientengruppe mit N=7 POD-Patienten/Patientinnen und N=7 nonPOD-Patienten/Patientinnen angegeben.

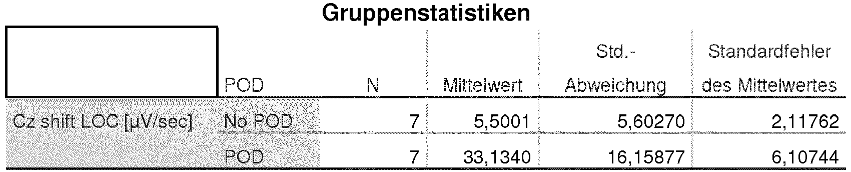

Die Signifikanz des Anstiegs der mittleren Amplitude von Zeitpunkt 1 zu Zeitpunkt 2 für die POD-Patienten/Patientinnen liegt bei 0,003 (Kruskal-Wallis-Test), d. h. es liegt eine deutliche Signifikanz vor.

Zum Zeitpunkt 3, d.h. zu einem intraoperativen Zeitpunkt nach dem Eintreten der narkoseinduzierten Bewusstlosigkeit ist die mittlere Amplitude sowohl bei den POD-Patienten/Patientinnen als auch bei den nonPOD-Patienten/Patientinnen wieder abgefallen. Ein Peak des Verlaufs liegt somit nur bei den POD-Patienten/Patientinnen vor. Nachfolgend sind die Werte einer Gruppenstatistik zum Zeitpunkt 3 für eine Patientengruppe mit N=7 POD-Patienten/Patientinnen und N=10 nonPOD-Patienten/Patientinnen angegeben.

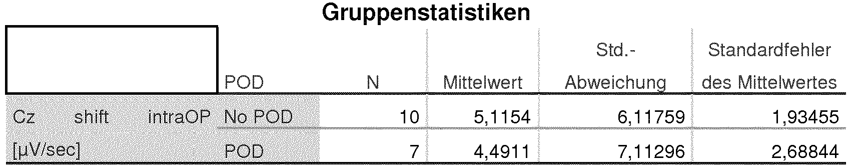

Bei den Messungen erfolgte wie erläutert die Messung des DC-EEG-Signals. Die Ableitung erfolgte mit der Elektrodenposition Cz (vgl. Figur 9). Die gleiche Dynamik zeigte sich jedoch über den gesamten Kortex, so dass auch eine frontale oder hoch frontale Ableitung erfolgen kann.

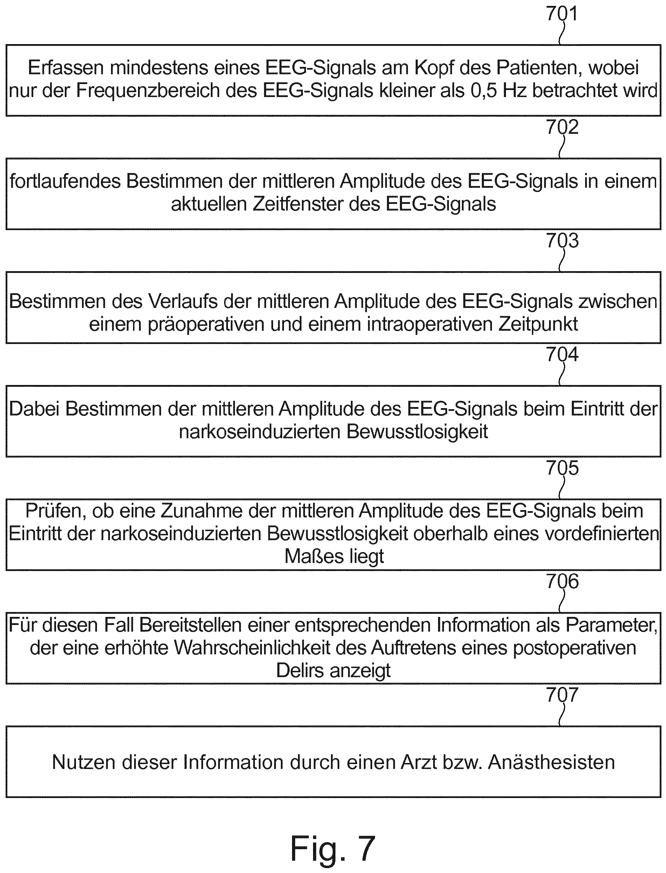

Die Figur 7 zeigt beispielhaft das zugehörige Verfahren zur Bestimmung eines Parameters, der auf eine erhöhte Wahrscheinlichkeit des Auftretens eines postoperativen Delirs hinweist. Gemäß Schritt 701 wird mindestens ein EEG-Signal am Kopf des/r Patienten/Patientin erfasst, wobei nur der Frequenzbereich des EEG-Signals kleiner als 0,5 Hz betrachtet wird, also das Direct Current EEG-Signal oder DC-EEG-Signal. Beispielsweise erfolgt eine Ableitung über eine Elektrode an der Position Cz und eine Referenzelektrode beispielsweise am Ohr.

Gemäß Schritt 702 wird fortlaufend die mittlere Amplitude des EEG-Signals in einem aktuellen Zeitfenster des EEG-Signals bestimmt. Diese fortlaufende Bestimmung der mittleren Amplitude erlaubt die Bestimmung des Verlaufs der mittleren Amplitude des EEG-Signals zwischen einem präoperativen und einem intraoperativen Zeitpunkt gemäß Schritt 703. Dabei wird in Schritt 704 die mittlere Amplitude des EEG Signals beim Eintritt der narkoseinduzierten Bewusstlosigkeit bestimmt.

In Schritt 705 wird geprüft, ob eine Zunahme der mittleren Amplitude des EEG Signals beim Eintritt der narkoseinduzierten Bewusstlosigkeit erfolgte und, wenn ja, ob die Zunahme oberhalb eines vordefinierten Maßes liegt. Das vordefinierte Maß wird dabei beispielsweise über zwei Referenzwerte ermittelt, wobei der erste Referenzwert die mittlere Amplitude des EEG-Signals beim Eintritt der narkoseinduzierten Bewusstlosigkeit bei einer Mehrzahl von nonPOD-Patienten/Patientinnen angibt und der zweite Referenzwert die mittlere Amplitude des EEG-Signals beim Eintritt der narkoseinduzierten Bewusstlosigkeit bei einer Mehrzahl von POD-Patienten/Patientinnen angibt. Diese Referenzwerte wurden zuvor an Gruppen von POD-Patienten/Patientinnen und nonPOD-Patienten/Patientinnen bestimmt.

Wenn beispielsweise der zweite Referenzwert um einen bestimmten Faktor, beispielsweise um den Faktor 3 höher ist als der erste Referenzwert, so ist das vordefinierte Maß überschritten.

Für diesen Fall wird in Schritt 706 eine entsprechende Information als Parameter bereitgestellt, der das Eintreten eines postoperativen Delirs indiziell anzeigt. Diese Information wird dann durch eine/n Arzt/Ärztin oder Anästhesisten/Anästhesistin in Schritt 707 genutzt. Beispielsweise kann der/die Arzt/Ärztin oder Anästhesist/Anästhesistin diese Information dazu nutzen, abhängig von weiteren Parametern des/r Patienten/Patientin die Narkose weniger tief durchzuführen und/oder unmittelbar nach der Narkose unterstützende therapeutische Maßnahmen einzuleiten, die der Entwicklung eines postoperativen Delirs entgegenwirken.

Zur Durchführung der Verfahren gemäß den Figuren 4, 5 und 7 kann ein EEG-basierter Gehirnfunktions-Monitor oder allgemein ein Computer eingesetzt werden. Die Verfahrensschritte zur Bestimmung der intraoperativen Alpha-Peak Frequenz und zur Vornahme des Vergleichs mit dem Referenzwert (Figur 3) bzw. zur Bestimmung der Leistung des Alpha-Bands des EEG-Signals und zur Vornahme der Prüfung, ob eine Zunahme der Leistung des Alpha-Bands von der ersten Leistung zu der zweiten Leistung unterhalb eines vordefinierten Maßes liegt, bzw. zur Bestimmung des Verlaufs der mittleren Amplitude des "Direct Current" EEG-Signals und Auswertung des Verlaufs werden dabei durch einen Programmcode ausgeführt, der in einem Prozessor ausgeführt wird. Der Programmcode ist im einem Speicher des Prozessors gespeichert bzw. wird vor der Ausführung in diesen geladen. Bei dem Prozessor, der den Programmcode ausführt, kann es sich um den Hauptprozessor des EEG-Monitors handeln oder um einen gesonderten Prozessor.

Die Figur 7 zeigt beispielhaft eine mögliche Implementierung eines solchen EEG-basierten Gehirnfunktions-Monitors 1. Der EEG-Monitor 1 umfasst einen Mikroprozessor 2, einen Speicher 3, eine Steuereinrichtung 4, eine Ausgabeeinheit 5 und eine Schnittstelle 7 zum Anschluss von EEG-Kabeln.

Über die Schnittstelle 7 können EEG-Kabel mit EEG-Elektroden 61, 62 an den EEG-Monitor 1 angeschlossen werden. Es sind beispielhaft zwei EEG-Kabel dargestellt, die ein EEG-Signal aufnehmen, wobei weitere EEG-Kabel zur Aufnahme eines mehrkanaligen EEG-Signals vorgesehen sein können.

Das EEG-Signal wird dem Mikroprozessor 2 zugeführt. Im Speicher 3 ist der Programmcode gespeichert oder es kann ein Programmcode in den Speicher 3 geladen werden, der bei Ausführung im Mikroprozessor 2 das in Bezug auf die Figur 4 und/oder das in Bezug auf die Figur 5 und/oder das in Bezug auf die Figur 7 erläuterte Verfahren ausführt. Über die Steuereinrichtung 4 kann der Ablauf gesteuert werden und diese kann dazu eingerichtet sein, entsprechende Eingabebefehler zu erhalten. Die Steuereinrichtung 4 kann dabei ein Hauptprozessor des EEG-Monitors 1 sein oder einen solchen enthalten. Alternativ kann die Funktionalität des Mikroprozessors 2 durch die Steuereinrichtung 4 übernommen werden. Über die Steuereinrichtung 4 und/oder weitere, nicht dargestellte Module können dabei weitere Funktionalitäten des EEG-Monitors 1 realisiert werden.

Der Mikroprozessor 2 bestimmt somit bei Ausführung des geladenen Programmcodes die intraoperative Alpha-Peak Frequenz, vergleicht diese mit einem Referenzwert und bestimmt, ob die bestimmte intraoperative Alpha-Peak Frequenz signifikant niedriger liegt als ein vorgegebener Referenzwert der Alpha-Peak Frequenz. Die entsprechende Information wird an die Ausgabeeinheit 5 übertragen und an dieser ausgegeben. Dies kann beispielsweise über einen Monitor 51 und/oder eine akustische Einheit 52 erfolgen.

Alternativ oder ergänzend wertet der Mikroprozessor 2 bei Ausführung des geladenen Programmcodes die Leistung im Alpha-Band gemäß der Figur 5 aus. Dabei wird geprüft, ob eine Zunahme der Leistung des Alpha-Bands von der Leistung präoperativ zu der Leistung intraoperativ unterhalb eines vordefinierten Maßes liegt. Die entsprechende Information wird an die Ausgabeeinheit 5 übertragen und an dieser ausgegeben. Dies kann über den Monitor 51 und/oder die akustische Einheit 52 erfolgen.

Alternativ oder ergänzend wertet der Mikroprozessor 2 bei Ausführung des geladenen Programmcodes den Verlauf der mittleren Amplitude des "Direct Current" EEG-Signals gemäß der Figur 7 aus. Dabei wird geprüft, ob die Zunahme der mittleren Amplitude des EEG-Signals beim Eintritt der narkoseinduzierten Bewusstlosigkeit oberhalb eines vordefinierten Maßes liegt. Die entsprechende Information wird an die Ausgabeeinheit 5 übertragen und an dieser ausgegeben. Dies kann über den Monitor 51 und/oder die akustische Einheit 52 erfolgen.

Es versteht sich, dass die Erfindung nicht auf die oben beschriebenen Ausführungsformen beschränkt ist und verschiedene Modifikationen und Verbesserungen vorgenommen werden können, ohne von den hier beschriebenen Konzepten abzuweichen. Beliebige der Merkmale können separat oder in Kombination mit beliebigen anderen Merkmalen eingesetzt werden, sofern sie sich nicht gegenseitig ausschließen, und die Offenbarung dehnt sich auf alle Kombinationen und Unterkombinationen eines oder mehrerer Merkmale, die hier beschrieben werden, aus und umfasst diese. Soweit Bereiche definiert sind, so umfassen diese sämtliche Werte innerhalb dieser Bereiche sowie sämtliche Teilbereiche, die in einen Bereich fallen.

## Patentansprüche

1. Vorrichtung zur Bereitstellung eines Parameters, der auf eine erhöhte Wahrscheinlichkeit des Auftretens eines postoperativen Delirs hinweist, wobei die Vorrichtung aufweist:
- Mittel (2), die dazu vorgesehen und eingerichtet sind, mindestens ein EEG-Signal am Kopf des/r Patienten/Patientin zu erfassen,
- Mittel (2), die dazu vorgesehen und eingerichtet sind, die intraoperative Alpha- Peak Frequenz des EEG-Signals zu bestimmen, wobei die Alpha-Peak Frequenz im Leistungsspektrum des EEG-Signals die Frequenz im Alpha-Band ist, bei der die Leistung am größten ist,
- Mittel (2), die dazu vorgesehen und eingerichtet sind zu prüfen, ob die bestimmte intraoperative Alpha-Peak Frequenz signifikant niedriger liegt als ein vorgegebener Referenzwert der Alpha-Peak Frequenz,
- Mittel (2), die dazu vorgesehen und eingerichtet sind, eine entsprechende Information als Parameter, der eine erhöhte Wahrscheinlichkeit des Auftretens eines postoperativen Delirs anzeigt, bereitzustellen.

2. Vorrichtung nach Anspruch 1, **dadurch gekennzeichnet, dass** der vorgegebene Referenzwert ermittelt worden ist durch Mittelung der gemessenen intraoperativen Alpha-Peak Frequenzen einer Mehrzahl von Patienten/Patientinnen, die kein postoperatives Delir entwickelt haben und/oder **dadurch gekennzeichnet, dass** der vorgegebene Referenzwert abhängig ist von der Altersklasse, in der der/die Patient/Patientin sich befindet.

3. Vorrichtung nach einem der Ansprüche 1 oder 2, **dadurch gekennzeichnet, dass** die Mittel (2) dazu vorgesehen und eingerichtet sind, die intraoperativen Alpha-Peak Frequenz zu einem Zeitpunkt zu bestimmen, zu dem der/die Patient/Patientin sich in einer stabilen Narkose befindet.

4. Vorrichtung nach einem der Ansprüche 1 bis 3, **dadurch gekennzeichnet, dass** die Mittel (2) dazu vorgesehen und eingerichtet sind, festzustellen, dass die intraoperative Alpha-Peak Frequenz dann signifikant niedriger liegt als ein vorgegebener Referenzwert der Alpha-Peak Frequenz, wenn die Differenz zwischen dem vorgegebenen Referenzwert und der intraoperative Alpha-Peak Frequenz eine definierte prozentuale Abweichung vom Referenzwert oder eine definierte absolute Differenz zwischen der intraoperativen Alpha-Peak Frequenz und dem Referenzwert übersteigt.

5. Vorrichtung nach einem der Ansprüche 1 bis 4, **dadurch gekennzeichnet, dass** die Mittel (2) dazu vorgesehen und eingerichtet sind, mindestens ein frontales EEG- Signal am Kopf des/r Patienten/Patientin zu erfassen und/oder **dadurch gekennzeichnet, dass** die Mittel (2) dazu vorgesehen und eingerichtet sind, mehrere frontale EEG-Signale des Patienten zu erfassen, die zur Bestimmung der Alpha-Peak Frequenz gemittelt werden und/oder **dadurch gekennzeichnet, dass** die Mittel (2) dazu vorgesehen und eingerichtet sind, die Alpha-Peak Frequenz an dem EEG-Signal zu bestimmen, nachdem dieses durch einen Bandpass-Filter gefiltert worden ist.

6. Vorrichtung nach einem der Ansprüche 1 bis 5, **dadurch gekennzeichnet, dass** des Weiteren
- die Mittel (2) dazu vorgesehen und eingerichtet sind, die Leistung des Alpha- Bands des EEG-Signals zu bestimmen, wobei die Leistung des Alpha-Bands im Leistungsspektrum des EEG-Signals definiert ist als Integral der Leistung über alle Frequenzen im Alpha-Band, wobei
- die Mittel (2) dazu vorgesehen und eingerichtet sind, eine erste Leistung des Alpha-Bands zu einem präoperativen Zeitpunkt zu bestimmen, der vor der Gabe eines narkoseinduzierenden Medikaments liegt, und eine zweite Leistung des Alpha-Bands zu einem intraoperativen Zeitpunkt zu bestimmen, der nach dem Eintreten der narkoseinduzierten Bewusstlosigkeit liegt, die Mittel (2) dazu vorgesehen und eingerichtet sind zu prüfen, ob eine Zunahme der Leistung des Alpha-Bands von der ersten Leistung zu der zweiten Leistung unterhalb eines vordefinierten Maßes liegt,
die Mittel (2) dazu vorgesehen und eingerichtet sind, für diesen Fall eine entsprechende Information als Parameter, der eine erhöhte Wahrscheinlichkeit des Auftretens eines postoperativen Delirs anzeigt, bereitzustellen.

7. Vorrichtung zur Bereitstellung eines Parameters, der auf eine erhöhte Wahrscheinlichkeit des Auftretens eines postoperativen Delirs hinweist, wobei die Vorrichtung aufweist:
- Mittel (2), die dazu vorgesehen und eingerichtet sind, mindestens ein EEG-Signal am Kopf des Patienten zu erfassen,
- Mittel (2), die dazu vorgesehen und eingerichtet sind, die Leistung des Alpha- Bands des EEG-Signals zu bestimmen, wobei die Leistung des Alpha-Bands im Leistungsspektrum des EEG-Signals definiert ist als Integral der Leistung über alle Frequenzen im Alpha-Band,
- wobei eine erste Leistung des Alpha-Bands bestimmt wird zu einem präoperativen Zeitpunkt, der vor der Gabe eines narkoseinduzierenden Medikaments liegt, und eine zweite Leistung des Alpha-Bands bestimmt wird zu einem intraoperativen Zeitpunkt, der nach dem Eintreten der narkoseinduzierten Bewusstlosigkeit liegt,
- Mittel (2), die dazu vorgesehen und eingerichtet sind zu prüfen, ob eine Zunahme der Leistung des Alpha-Bands von der ersten Leistung zu der zweiten Leistung unterhalb eines vordefinierten Maßes liegt,
- Mittel (2), die dazu vorgesehen und eingerichtet sind, eine entsprechende Information als Parameter, der eine erhöhte Wahrscheinlichkeit des Auftretens eines postoperativen Delirs anzeigt, bereitzustellen.

8. Vorrichtung nach Anspruch 7, **dadurch gekennzeichnet, dass** das vordefinierte Maß, unterhalb dessen eine Zunahme der Leistung des Alpha-Bands von der ersten Leistung zu der zweiten Leistung liegen muss, damit die Information bereitgestellt wird, durch die Mittel (2) dadurch bestimmt wird, dass
für eine Mehrzahl von Patienten/Patientinnen, die kein postoperatives Delir entwickelt haben, die gemittelte erste Leistung des Alpha-Bands mit der gemittelten zweiten Leistung des Alpha-Bands verglichen wird, wobei ein erster Referenzwert gebildet wird, für eine Mehrzahl von Patienten/Patientinnen, die ein postoperatives Delir entwickelt haben, die gemittelte erste Leistung des Alpha-Bands mit der gemittelten zweiten Leistung des Alpha-Bands verglichen wird, wobei ein zweiter Referenzwert gebildet wird,
das vordefinierte Maß auf der Grundlage der Differenz zwischen dem ersten Referenzwert und dem zweiten Referenzwert gebildet wird.

9. Vorrichtung nach Anspruch 8, **dadurch gekennzeichnet, dass** das vordefinierte Maß durch die Differenz zwischen dem ersten Referenzwert und dem zweiten Referenzwert abzüglich eines prozentualen oder absoluten Toleranzwertes gebildet wird und/oder **dadurch gekennzeichnet, dass** das vordefinierte Maß abhängig von der Altersklasse, in der der/die Patient/Patientin sich befindet, bestimmt wird.

10. Vorrichtung nach einem der Ansprüche 7 bis 10, **dadurch gekennzeichnet, dass** die erste Leistung des Alpha-Bands und die zweite Leistung des Alpha-Bands sowie die Zunahme der Leistung des Alpha-Bands von der ersten Leistung zu der zweiten Leistung in dB bestimmt wird, und das Maß, unterhalb dessen eine Zunahme der Leistung des Alpha-Bands von der ersten Leistung zu der zweiten Leistung liegen muss, damit die Information bereitgestellt wird und geprüft wird, ebenfalls in dB angegeben ist und/oder **dadurch gekennzeichnet, dass** die Zunahme der Leistung des Alpha-Bands von der ersten Leistung zu der zweiten Leistung unterhalb von 15 dB, insbesondere unterhalb von 12 dB liegt, damit die Information durch die Mittel (2) bereitgestellt wird und/oder **dadurch gekennzeichnet, dass** die Mittel (2) dazu vorgesehen und eingerichtet sind, die Leistung des Alpha-Bands an dem EEG-Signal zu bestimmen, nachdem dieses durch einen Bandpass-Filter gefiltert worden ist und/oder **dadurch gekennzeichnet, dass** die Mittel (2) dazu vorgesehen und eingerichtet sind, mindestens ein frontales EEG- Signal am Kopf des/r Patienten/Patientin zu erfassen und/oder **dadurch gekennzeichnet, dass** die Mittel (2) dazu vorgesehen und eingerichtet sind, im 10-20-System Signale von Elektroden abzuleiten, die an den Positionen F7, F8, Fp1, Fp2 und Fpz positioniert sind.

11. Vorrichtung zur Bereitstellung eines Parameters, der auf eine erhöhte Wahrscheinlichkeit des Auftretens eines postoperativen Delirs hinweist, wobei die Vorrichtung aufweist:
- Mittel (2), die dazu vorgesehen und eingerichtet sind, mindestens ein EEG-Signal am Kopf des/r Patienten/Patientin zu erfassen, wobei nur der Frequenzbereich des EEG-Signals kleiner als 0,5 Hz betrachtet wird,
- Mittel (2), die dazu vorgesehen und eingerichtet sind, die mittlere Amplitude des EEG-Signals in einem aktuellen Zeitfenster des EEG-Signals zu bestimmen,
- Mittel (2), die dazu vorgesehen und eingerichtet sind, den Verlauf der mittleren Amplitude des EEG-Signals zwischen einem präoperativen Zeitpunkt, der vor der Gabe eines narkoseinduzierenden Medikaments liegt, und einem intraoperativen Zeitpunkt, der nach dem Eintreten der narkoseinduzierten Bewusstlosigkeit liegt, zu bestimmen,
- wobei die mittlere Amplitude des EEG-Signals beim Eintritt der narkoseinduzierten Bewusstlosigkeit bestimmt wird,
- Mittel (2), die dazu vorgesehen und eingerichtet sind, zu prüfen, ob eine Zunahme der mittleren Amplitude des EEG-Signals beim Eintritt der narkoseinduzierten Bewusstlosigkeit oberhalb eines vordefinierten Maßes liegt, und
- Mittel (2), die dazu vorgesehen und eingerichtet sind, für diesen Fall eine entsprechende Information als Parameter, der eine erhöhte Wahrscheinlichkeit des Auftretens eines postoperativen Delirs anzeigt, bereitzustellen.

12. Vorrichtung nach Anspruch 11, **dadurch gekennzeichnet, dass** die Mittel (2) die mittlere Amplitude des EEG-Signals dahingehend fortlaufend bestimmen, als sie mindestens alle 120 Sekunden, insbesondere mindestens alle 60 Sekunden, insbesondere mindestens alle 30 Sekunden, insbesondere mindestens alle 10 Sekunden, insbesondere mindestens alle 2 Sekunden neu bestimmt wird und/oder **dadurch gekennzeichnet, dass** ein Tiefpassfilter mit einer oberen Eckfrequenz von 0,5 Hz vorgesehen ist, wobei das EEG-Signal den Tiefpassfilter durchläuft.

13. Vorrichtung nach einem der Ansprüche 11 oder 12, **dadurch gekennzeichnet, dass** das vordefinierte Maß, oberhalb dessen eine Zunahme der mittleren Amplitude des EEG-Signals beim Eintritt der narkoseinduzierten Bewusstlosigkeit liegen muss, damit eine Information bereitgestellt wird, dadurch bestimmt wird, dass
für eine Mehrzahl von Patienten/Patientinnen, die kein postoperatives Delir entwickelt haben, die mittleren Amplitude des EEG-Signals beim Eintritt der narkoseinduzierten Bewusstlosigkeit bestimmt wird, wobei ein erster Referenzwert gebildet wird,
für eine Mehrzahl von Patienten/Patientinnen, die ein postoperatives Delir entwickelt haben, die mittlere Amplitude des EEG-Signals beim Eintritt der narkoseinduzierten Bewusstlosigkeit bestimmt wird, wobei ein zweiter Referenzwert gebildet wird,
das vordefinierte Maß auf der Grundlage der Differenz oder eines Verhältnisses zwischen dem ersten Referenzwert und dem zweiten Referenzwert gebildet wird.

14. Vorrichtung nach Anspruch 13, **dadurch gekennzeichnet, dass** der zweite Referenzwert um mindestens den Faktor 3, insbesondere um mindestens den Faktor 5, insbesondere um mindestens den Faktor 10 größer ist als der erste Referenzwert.

15. EEG Narkosemonitor (1) mit einer Vorrichtung nach Anspruch 1 und/oder mit einer Vorrichtung nach Anspruch 7 und/oder mit einer Vorrichtung nach Anspruch 11.

## Claims

1. Device for providing a parameter that indicates an increased likelihood of postoperative delirium occurring, wherein the device comprises:
- means (2) provided and designed to record at least one EEG signal at the head of the patient,
- means (2) provided and designed to determine the intraoperative alpha peak frequency of the EEG signal, wherein the alpha peak frequency in the power spectrum of the EEG signal is the frequency in the alpha band at which the power is greatest,
- means (2) provided and designed to check whether the determined intraoperative alpha peak frequency is significantly lower than a specified reference value of the alpha peak frequency,
- means (2) provided and designed to provide corresponding information as a parameter that indicates an increased likelihood of postoperative delirium occurring.

2. Device according to claim 1, **characterized in that** the specified reference value has been determined by averaging the measured intraoperative alpha peak frequencies of a plurality of patients who have not developed postoperative delirium, and/or **characterized in that** the specified reference value depends on the age group to which the patient belongs.

3. Device according to either claim 1 or claim 2, **characterized in that** the means (2) are provided and designed to determine the intraoperative alpha peak frequency at a time when the patient is under stable anesthesia.

4. Device according to any of claims 1 to 3, **characterized in that** the means (2) are provided and designed to determine that the intraoperative alpha peak frequency is significantly lower than a specified reference value of the alpha peak frequency if the difference between the specified reference value and the intraoperative alpha peak frequency exceeds a defined percentage deviation from the reference value or a defined absolute difference between the intraoperative alpha peak frequency and the reference value.

5. Device according to any of claims 1 to 4, **characterized in that** the means (2) are provided and designed to record at least one frontal EEG signal at the head of the patient, and/or **characterized in that** the means (2) are provided and designed to record a plurality of frontal EEG signals of the patient, which are averaged to determine the alpha peak frequency, and/or **characterized in that** the means (2) are provided and designed to determine the alpha peak frequency of the EEG signal after it has been filtered by a bandpass filter.

6. Device according to any of claims 1 to 5, **characterized in that,** furthermore,
- the means (2) are provided and designed to determine the power of the alpha band of the EEG signal, wherein the power of the alpha band in the power spectrum of the EEG signal is defined as the integral of the power over all frequencies in the alpha band, wherein
- the means (2) are provided and designed to determine a first power of the alpha band at a preoperative time prior to the administration of an anesthetic-inducing drug, and to determine a second power of the alpha band at an intraoperative time after the onset of the anesthetic-induced loss of consciousness, the means (2) are provided and designed to check whether an increase in the power of the alpha band from the first power to the second power is below a predefined amount,
the means (2) are provided and designed to provide corresponding information in this case, as a parameter that indicates an increased likelihood of postoperative delirium occurring.

7. Device for providing a parameter that indicates an increased likelihood of postoperative delirium occurring, wherein the device comprises:
- means (2) provided and designed to detect at least one EEG signal at the head of the patient,
- means (2) provided and designed to determine the power of the alpha band of the EEG signal, wherein the power of the alpha band in the power spectrum of the EEG signal is defined as the integral of the power over all frequencies in the alpha band,
- wherein a first power of the alpha band is determined at a preoperative time prior to the administration of an anesthetic-inducing drug, and a second power of the alpha band is determined at an intraoperative time after the onset of the anesthetic-induced loss of consciousness,
- means (2) provided and designed to check whether an increase in the power of the alpha band from the first power to the second power is below a predefined amount,
- means (2) provided and designed to provide corresponding information as a parameter that indicates an increased likelihood of postoperative delirium occurring.

8. Device according to claim 7, **characterized in that** the predefined amount, below which an increase in the power of the alpha band from the first power to the second power must be in order for the information to be provided, is determined by the means (2) **in that**
for a plurality of patients who have not developed postoperative delirium, the averaged first power of the alpha band is compared with the averaged second power of the alpha band, wherein a first reference value is formed, for a plurality of patients who have developed postoperative delirium, the averaged first power of the alpha band is compared with the averaged second power of the alpha band, wherein a second reference value is formed,
the predefined amount is formed on the basis of the difference between the first reference value and the second reference value.

9. Device according to claim 8, **characterized in that** the predefined amount is formed by the difference between the first reference value and the second reference value minus a percentage or absolute tolerance value, and/or **characterized in that** the predefined amount is determined depending on the age group to which the patient belongs.

10. Device according to any of claims 7 to 10, **characterized in that** the first power of the alpha band and the second power of the alpha band and the increase in the power of the alpha band from the first power to the second power are determined in dB, and the amount, below which an increase in the power of the alpha band from the first power to the second power must be for the information to be provided and checked, is also specified in dB, and/or **characterized in that** the increase in the power of the alpha band from the first power to the second power is below 15 dB, in particular below 12 dB, so that the information is provided by the means (2), and/or **characterized in that** the means (2) are provided and designed to determine the power of the alpha band of the EEG signal after it has been filtered by a bandpass filter, and/or **characterized in that** the means (2) are provided and designed to record at least one frontal EEG signal at the head of the patient, and/or **characterized in that** the means (2) are provided and designed to derive signals from electrodes positioned at positions F7, F8, Fp1, Fp2 and Fpz in the 10-20 system.

11. Device for providing a parameter that indicates an increased likelihood of postoperative delirium occurring, wherein the device comprises:
- means (2) provided and designed to detect at least one EEG signal at the head of the patient, wherein only the frequency range of the EEG signal below 0.5 Hz is taken into account,
- means (2) provided and designed to determine the mean amplitude of the EEG signal in a current time window of the EEG signal,
- means (2) provided and designed to determine the course of the mean amplitude of the EEG signal between a preoperative time prior to the administration of an anesthetic-inducing drug and an intraoperative time after the onset of the anesthetic-induced loss of consciousness,
- wherein the mean amplitude of the EEG signal is determined at the onset of anesthesia-induced loss of consciousness,
- means (2) provided and designed to check whether an increase in the mean amplitude of the EEG signal at the onset of anesthetic-induced loss of consciousness is above a predefined amount, and
- means (2) provided and designed to provide corresponding information in this case, as a parameter that indicates an increased likelihood of postoperative delirium occurring.

12. Device according to claim 11, **characterized in that** the means (2) continuously determine the mean amplitude of the EEG signal in such a way that it is re-determined at least every 120 seconds, in particular at least every 60 seconds, in particular at least every 30 seconds, in particular at least every 10 seconds, in particular at least every 2 seconds, and/or **characterized in that** a low-pass filter with an upper cut-off frequency of 0.5 Hz is provided, wherein the EEG signal passes through the low-pass filter.

13. Device according to either of claims 11 or 12, **characterized in that** the predefined amount, above which an increase in the mean amplitude of the EEG signal at the onset of anesthesia-induced loss of consciousness must be in order for information to be provided, is determined **in that**
for a plurality of patients who have not developed postoperative delirium, the mean amplitude of the EEG signal at the onset of anesthesia-induced loss of consciousness is determined, wherein a first reference value is formed,
for a plurality of patients who have developed postoperative delirium, the mean amplitude of the EEG signal at the onset of anesthesia-induced loss of consciousness is determined, wherein a second reference value is formed,
the predefined amount is formed on the basis of the difference or a ratio between the first reference value and the second reference value.

14. Device according to claim 13, **characterized in that** the second reference value is greater than the first reference value by at least a factor of 3, in particular at least a factor of 5, in particular at least a factor of 10.

15. EEG anesthesia monitor (1) comprising a device according to claim 1 and/or comprising a device according to claim 7 and/or comprising a device according to claim 11.

## Revendications

1. Dispositif destiné à fournir un paramètre qui indique une probabilité accrue de survenue d'un délirium postopératoire, dans lequel le dispositif présente :
- des moyens (2) qui sont prévus et configurés pour détecter au moins un signal EEG au niveau de la tête du/de la patient(e),
- des moyens (2) qui sont prévus et configurés pour déterminer la fréquence de pic alpha peropératoire du signal EEG, dans lequel la fréquence de pic alpha dans le spectre de puissance du signal EEG est la fréquence dans la bande alpha à laquelle la puissance est la plus élevée,
- des moyens (2) qui sont prévus et configurés pour vérifier si la fréquence de pic alpha peropératoire déterminée est significativement inférieure à une valeur de référence prédéfinie de la fréquence de pic alpha,
- des moyens (2) qui sont prévus et configurés pour fournir une information correspondante en tant que paramètre qui indique une probabilité accrue de survenue d'un délirium postopératoire.

2. Dispositif selon la revendication 1, **caractérisé en ce que** la valeur de référence prédéfinie a été déterminée en faisant la moyenne des fréquences de pic alpha peropératoires mesurées d'une pluralité de patient(e)s qui n'ont pas développé de délirium postopératoire et/ou **caractérisé en ce que** la valeur de référence prédéfinie dépend de la classe d'âge dans laquelle se trouve le/la patient(e).

3. Dispositif selon l'une des revendications 1 ou 2, **caractérisé en ce que** les moyens (2) sont prévus et configurés pour déterminer la fréquence de pic alpha peropératoire à un moment où le/la patient(e) est dans un état d'anesthésie stable.

4. Dispositif selon l'une des revendications 1 à 3, **caractérisé en ce que** les moyens (2) sont prévus et configurés pour constater que la fréquence de pic alpha peropératoire est significativement inférieure à une valeur de référence prédéfinie de la fréquence de pic alpha lorsque la différence entre la valeur de référence prédéfinie et la fréquence de pic alpha peropératoire dépasse un écart en pourcentage défini par rapport à la valeur de référence ou une différence absolue définie entre la fréquence de pic alpha peropératoire et la valeur de référence.

5. Dispositif selon l'une des revendications 1 à 4, **caractérisé en ce que** les moyens (2) sont prévus et configurés pour détecter au moins un signal EEG frontal au niveau de la tête du/de la patient(e) et/ou **caractérisé en ce que** les moyens (2) sont prévus et configurés pour détecter plusieurs signaux EEG frontaux du patient qui sont moyennés pour déterminer la fréquence de pic alpha et/ou **caractérisé en ce que** les moyens (2) sont prévus et configurés pour déterminer la fréquence de pic alpha sur le signal EEG après que celui-ci a été filtré par un filtre passe-bande.

6. Dispositif selon l'une des revendications 1 à 5, **caractérisé en ce qu'**en outre
- les moyens (2) sont prévus et configurés pour déterminer la puissance de la bande alpha du signal EEG, dans lequel la puissance de la bande alpha dans le spectre de puissance du signal EEG est définie comme l'intégrale de la puissance sur toutes les fréquences dans la bande alpha, dans lequel
- les moyens (2) sont prévus et configurés pour déterminer une première puissance de la bande alpha à un moment préopératoire qui se situe avant l'administration d'un médicament induisant une anesthésie, et pour déterminer une seconde puissance de la bande alpha à un moment peropératoire qui se situe après l'apparition de la perte de conscience induite par l'anesthésie, les moyens (2) sont prévus et configurés pour vérifier si une augmentation de la puissance de la bande alpha de la première puissance à la seconde puissance est inférieure à une mesure prédéfinie,
les moyens (2) sont prévus et configurés pour fournir, dans ce cas, une information correspondante en tant que paramètre qui indique une probabilité accrue de survenue d'un délirium postopératoire.

7. Dispositif destiné à fournir un paramètre qui indique une probabilité accrue de survenue d'un délirium postopératoire, dans lequel le dispositif présente :
- des moyens (2) qui sont prévus et configurés pour détecter au moins un signal EEG au niveau de la tête du patient,
- des moyens (2) qui sont prévus et configurés pour déterminer la puissance de la bande alpha du signal EEG, dans lequel la puissance de la bande alpha dans le spectre de puissance du signal EEG est définie comme l'intégrale de la puissance sur toutes les fréquences dans la bande alpha,
- dans lequel une première puissance de la bande alpha est déterminée à un moment préopératoire qui se situe avant l'administration d'un médicament induisant une anesthésie, et une seconde puissance de la bande alpha est déterminée à un moment peropératoire qui se situe après l'apparition de la perte de conscience induite par l'anesthésie,
- des moyens (2) qui sont prévus et configurés pour vérifier si une augmentation de la puissance de la bande alpha de la première puissance à la seconde puissance est inférieure à une mesure prédéfinie,
- des moyens (2) qui sont prévus et configurés pour fournir une information correspondante en tant que paramètre qui indique une probabilité accrue de survenue d'un délirium postopératoire.

8. Dispositif selon la revendication 7, **caractérisé en ce que** la mesure prédéfinie en dessous de laquelle doit se situer une augmentation de la puissance de la bande alpha de la première puissance à la seconde puissance pour que l'information soit fournie est déterminée par les moyens (2) par le fait que
pour une pluralité de patient(e)s qui n'ont pas développé de délirium postopératoire, la première puissance moyenne de la bande alpha est comparée avec la seconde puissance moyenne de la bande alpha, dans lequel une première valeur de référence est établie, pour une pluralité de patient(e)s qui ont développé un délirium postopératoire, la première puissance moyenne de la bande alpha est comparée avec la seconde puissance moyenne de la bande alpha, dans lequel une seconde valeur de référence est établie,
la mesure prédéfinie est établie sur la base de la différence entre la première valeur de référence et la seconde valeur de référence.

9. Dispositif selon la revendication 8, **caractérisé en ce que** la mesure prédéfinie est établie par la différence entre la première valeur de référence et la seconde valeur de référence moins une valeur de tolérance en pourcentage ou absolue et/ou **caractérisé en ce que** la mesure prédéfinie est déterminée en fonction de la classe d'âge dans laquelle se trouve le/la patient(e).

10. Dispositif selon l'une des revendications 7 à 10, **caractérisé en ce que** la première puissance de la bande alpha et la seconde puissance de la bande alpha ainsi que l'augmentation de la puissance de la bande alpha de la première puissance à la seconde puissance sont déterminées en dB, et la mesure en dessous de laquelle doit se situer une augmentation de la puissance de la bande alpha de la première puissance à la seconde puissance pour que l'information soit fournie et vérifiée est également indiquée en dB et/ou **caractérisé en ce que** l'augmentation de la puissance de la bande alpha de la première puissance à la seconde puissance est inférieure à 15 dB, en particulier inférieure à 12 dB, pour que l'information soit fournie par les moyens (2), et/ou **caractérisé en ce que** les moyens (2) sont prévus et configurés pour déterminer la puissance de la bande alpha sur le signal EEG après que celui-ci a été filtré par un filtre passe-bande et/ou **caractérisé en ce que** les moyens (2) sont prévus et configurés pour détecter au moins un signal EEG frontal au niveau de la tête du/de la patient(e) et/ou **caractérisé en ce que** les moyens (2) sont prévus et configurés pour dériver, dans le système 10-20, des signaux d'électrodes qui sont positionnées aux positions F7, F8, Fp1, Fp2 et Fpz.

11. Dispositif permettant de fournir un paramètre qui indique une probabilité accrue de survenue d'un délirium postopératoire, dans lequel le dispositif présente :
- des moyens (2) qui sont prévus et configurés pour détecter au moins un signal EEG au niveau de la tête du/de la patient(e), dans lequel seule la plage de fréquences du signal EEG inférieure à 0,5 Hz est considérée,
- des moyens (2) qui sont prévus et configurés pour déterminer l'amplitude moyenne du signal EEG dans une fenêtre temporelle actuelle du signal EEG,
- des moyens (2) qui sont prévus et configurés pour déterminer l'évolution de l'amplitude moyenne du signal EEG entre un moment préopératoire qui se situe avant l'administration d'un médicament induisant une anesthésie et un moment peropératoire qui se situe après l'apparition de la perte de conscience induite par l'anesthésie,
- dans lequel l'amplitude moyenne du signal EEG lors de l'apparition de la perte de conscience induite par l'anesthésie est déterminée,
- des moyens (2) qui sont prévus et configurés pour vérifier si une augmentation de l'amplitude moyenne du signal EEG lors de l'apparition de la perte de conscience induite par l'anesthésie est supérieure à une mesure prédéfinie, et
- des moyens (2) qui sont prévus et configurés pour fournir, dans ce cas, une information correspondante en tant que paramètre qui indique une probabilité accrue de survenue d'un délirium postopératoire.

12. Dispositif selon la revendication 11, **caractérisé en ce que** les moyens (2) déterminent en continu l'amplitude moyenne du signal EEG en ce sens qu'elle est déterminée de nouveau au moins toutes les 120 secondes, en particulier au moins toutes les 60 secondes, en particulier au moins toutes les 30 secondes, en particulier au moins toutes les 10 secondes, en particulier au moins toutes les 2 secondes, et/ou **caractérisé en ce qu'**un filtre passe-bas avec une fréquence de coupure supérieure de 0,5 Hz est prévu, dans lequel le signal EEG traverse le filtre passe-bas.

13. Dispositif selon l'une des revendications 11 ou 12, **caractérisé en ce que** la mesure prédéfinie au-dessus de laquelle doit se situer une augmentation de l'amplitude moyenne du signal EEG lors de l'apparition de la perte de conscience induite par l'anesthésie pour qu'une information soit fournie est déterminée par le fait que
pour une pluralité de patient(e)s n'ayant pas développé de délirium postopératoire, l'amplitude moyenne du signal EEG lors de l'apparition de la perte de conscience induite par l'anesthésie est déterminée, dans lequel une première valeur de référence est établie,
pour une pluralité de patient(e)s ayant développé un délirium postopératoire, l'amplitude moyenne du signal EEG lors de l'apparition de la perte de conscience induite par l'anesthésie est déterminée, dans lequel une seconde valeur de référence est établie,
la mesure prédéfinie est établie sur la base de la différence ou d'un rapport entre la première valeur de référence et la seconde valeur de référence.

14. Dispositif selon la revendication 13, **caractérisé en ce que** la seconde valeur de référence est supérieure à la première valeur de référence d'au moins un facteur 3, en particulier d'au moins un facteur 5, en particulier d'au moins un facteur 10.

15. Moniteur d'anesthésie EEG (1) comportant un dispositif selon la revendication 1 et/ou comportant un dispositif selon la revendication 7 et/ou comportant un dispositif selon la revendication 11.
